# EUROPÄISCHE PATENTSCHRIFT

(11) **EP 3 319 638 B1**
(45) Veröffentlichungstag und Bekanntmachung des Hinweises auf die Patenterteilung: **04.12.2019**
(21) Anmeldenummer: 16733901.9
(22) Anmeldetag: 20.06.2016
(51) Int. Cl.: A61K 45/06, A61K 31/352, A61P 35/00

(54) **CINEOLHALTIGE ZUSAMMENSETZUNG ZUR BEHANDLUNG VON TUMOR- UND/ODER KREBSERKRANKUNGEN**
CINEOL-CONTAINING COMPOSITION FOR THE TREATMENT OF TUMOURS AND/OR CANCERS
COMPOSITION CONTENANT DU CINÉOL POUR LE TRAITEMENT DE MALADIES TUMORALES ET/OU CANCÉREUSES

(30) Priorität: 23.10.2015 DE 102015013668; 26.10.2015 DE 102015013725; 12.11.2015 DE 102015119552
(43) Veröffentlichungstag der Anmeldung: 16.05.2018
(73) Patentinhaber: Maria Clementine Martin Klosterfrau Vertriebsgesellschaft mbH, 50670 Köln (DE)
(72) Erfinder: UNKAUF, Markus, 80538 München (DE); WOLLENBERG, Barbara, 23566 Lübeck (DE); PRIES, Ralph, 23619 Rehhorst (DE)
(74) Vertreter: Von Rohr Patentanwälte Partnerschaft mbB
(86) Internationale Anmeldenummer: PCT/EP2016/064159
(87) Internationale Veröffentlichungsnummer: WO 2017/067676

(56) Entgegenhaltungen:
- WO-A2-2014/141038
- FR-A1- 2 956 817
- TRIPTI MISHRA ET AL: "Composition and in vitro cytotoxic activities of essential oil of Hedychium spicatum from different geographical regions of western Himalaya by principal components analysis", NATURAL PRODUCT RESEARCH, Bd. 30, Nr. 10, 21. Juli 2015 (2015-07-21) , Seiten 1224-1227, XP055296775, GB ISSN: 1478-6419, DOI: 10.1080/14786419.2015.1049176 & Tripti Mishra ET AL: "SUPPLEMENTARY MATERIAL Composition and in vitro cytotoxic activities of essential oil of Hedychium spicatum from different geographical region of Western Himalaya by principal component analysis", , 21. Juli 2015 (2015-07-21), Seiten 1-10, XP055296780, Gefunden im Internet: URL:http://www.tandfonline.com/doi/suppl/1 0.1080/14786419.2015.1049176/suppl_file/gn pl_a_1049176_sm3874.pdf [gefunden am 2016-08-22]

## Beschreibung

Die vorliegende Erfindung betrifft das technische (d.h. medizinisch-therapeutische) Gebiet der Behandlung von insbesondere malignen Tumorerkrankungen bzw. Karzinomerkrankungen des Kopf-Hals-Bereichs.

Insbesondere betrifft die vorliegende Erfindung eine cineolhaltige Zusammensetzung, insbesondere eine cineolhaltige pharmazeutische Zusammensetzung, welche sich zur Verwendung bei der prophylaktischen bzw. therapeutischen Behandlung von insbesondere malignen Tumorerkrankungen bzw. Karzinomerkrankungen des Kopf-Hals-Bereichs eignet. In diesem Zusammenhang betrifft die vorliegende Erfindung auch Cineol als solches, welches sich zur Verwendung im Rahmen der zuvor genannten prophylaktischen bzw. therapeutischen Behandlung eignet. Diesbezüglich betrifft die vorliegende Erfindung zudem die Verwendung einer Cineol als pharmazeutischen Wirkstoff enthaltenden Zusammensetzung zur prophylaktischen bzw. therapeutischen Behandlung der in Rede stehenden Tumor- bzw. Karzinomerkrankungen bzw. zur Herstellung eines Arzneimittels zu Zwecken der prophylaktischen bzw. therapeutischen Behandlung der zugrundeliegenden Erkrankungen.

Die vorliegende Erfindung betrifft gleichermaßen die Verwendung von Cineol als pharmazeutischer Wirkstoff zur prophylaktischen bzw. therapeutischen Behandlung der in Rede stehenden Erkrankungen bzw. zur Herstellung eines Arzneimittels zur prophylaktischen bzw. therapeutischen Behandlung von insbesondere malignen Tumor- bzw. Karzinomerkrankungen des Kopf-Hals-Bereichs. Insbesondere betrifft die vorliegende Erfindung auch ein Arzneimittel als solches sowie darüber hinaus auch eine Wirkstoffkombination, insbesondere in Form eines Kit (Kit-of-parts), zur prophylaktischen bzw. therapeutischen Behandlung der in Rede stehenden Erkrankungen.

Unter Tumorerkrankungen bzw. Karzinomerkrankungen des Kopf-Hals-Bereichs (synonym auch als Kopf-Hals-Karzinome bezeichnet) wird im Rahmen der vorliegenden Erfindung im Allgemeinen eine Gruppe von insbesondere bösartigen bzw. malignen Tumoren, die im Kopf-Hals-Bereich ihren Ursprung haben bzw. in diesem Bereich lokalisiert sind, verstanden. Insbesondere bilden sich die meisten Kopf-Hals-Karzinome bzw. -Tumoren in der Mundhöhle, Kehlkopf, Rachen sowie im Bereich der inneren Nase bzw. der Nasennebenhöhlen. Als Hauptursache für die Entstehung von Krebserkrankungen des Kopf-Hals-Bereichs gilt der Nikotinmissbrauch in Form von Tabakrauchen. Auch andere Arten von Tabakkonsum, übermäßiger Alkoholkonsum, Virusinfektionen, wie Infektionen mit HPV (Humane Papillomviren), schlechte Mundhygiene und häufiger Kontakt mit Schad- bzw. Giftstoffen, wie beispielsweise Asbest, erhöhen das Risiko, einen Kopf-Hals-Tumor zu entwickeln. Die Wahrscheinlichkeit zur Ausbildung der Krebserkrankung kann sich zudem durch den übermäßigen Konsum von Alkohol in Kombination mit Rauchen weiterführend erhöhen, da Alkohol im Allgemeinen als Lösungsmittel für die in Zigarettenrauch befindlichen Kanzerogene dient und somit zu einem vermehrten Kontakt mit den zugrundeliegenden Giftstoffen führen kann.

Unter dem Begriff Kopf-Hals-Tumor bzw. Kopf-Hals-Krebserkrankung können in diesem Zusammenhang eine Vielzahl von Krebserkrankungen verstanden werden, welche im Allgemeinen im Bereich von Kopf und Hals auftreten. Im Einzelnen können Kopf-Hals-Tumoren beispielsweise den Mund bzw. die Mundhöhle, hierbei insbesondere auch die Lippe, die Zunge, den Mundboden, das Zahnfleisch, den Gaumen sowie die Speicheldrüse betreffen. Zudem können von der Krebserkrankung auch der Rachen, der Kehlkopf, die innere Nase sowie die Nasennebenhöhlen und der äußere Hals betroffen sein. Die der Kopf-Hals-Krebserkrankung zugrundeliegende Tumorart ist dabei insbesondere davon abhängig, aus welcher Art Gewebe der Kopf-Hals-Tumor entsteht. Kopf-Hals-Tumoren treten im Allgemeinen in Form von Karzinomen auf, welche sich aus der Schleimhaut entwickeln; die Tumore können jedoch auch in Form von Lymphomen, welche ihren Ursprung in lymphatischem Gewebe haben, sowie in Form von Sarkomen auftreten, welche sich vom Binde- und Stützgewebe ableiten.

Bei den Tumorerkrankungen des Kopf-Hals-Bereichs handelt es sich oftmals um sogenannte Plattenepithelkarzinome, welche in diesem Zusammenhang auch als Kopf-Hals-Plattenepithel-Karzinome (HNSCC) bezeichnet werden. Für Kopf-Hals-Plattenepithel-Karzinome gilt im Speziellen, dass diese zu den häufigsten üblichen Tumoren weltweit gehören. In diesem Zusammenhang stellen Kopf-Hals-Plattenepithel-Karzinome eine der häufigsten üblichen soliden Neoplasien weltweit dar, deren Auftreten gleichermaßen in Zusammenhang mit Tabakrauch-Exposition sowie den Konsum von Alkohol steht.

Insbesondere handelt es sich in der Mehrzahl der zugrundeliegenden Tumoren, nämlich zu über 90 %, um sogenannte Plattenepithelkarzinome (*Epithelioma spinocellulare*)*.* Hierbei handelt es sich maßgeblich um von den Epithelien der Haut und insbesondere der Schleimhäute ausgehende maligne Tumore, welche verrukös und oftmals ulzerierend wachsen. Plattenepithelkarzinome leiten sich häufig von der Epidermis, Plattenepithel-tragenden Schleimhäuten oder metaplasierten Zylinderepithelien ab und stellen ein Malignom mit ungleich großen, atypischen, insbesondere in Strängen oder Nestern angeordneten Zellen dar, die gegebenenfalls durch Desmosomen verbunden sein können.

Für weitergehende Einzelheiten zu dem Krankheitsbild der Plattenepithelkarzinome kann beispielsweise auch verwiesen werden auf Pschyrembel, Medizinisches Wörterbuch, 257. Auflage, 1993, Verlag Walter de Gruyter, Stichwort "Plattenepithelkarzinom", sowie auf Roche Lexikon Medizin, 3. Auflage, 1993, Urban & Schwarzenberg Verlag, Stichwort "Plattenepithelkarzinom".

In Deutschland entwickeln schätzungsweise 50 von 100.000 Menschen jährlich einen Kopf-Hals-Tumor, wobei mit großer Häufigkeit der Mundhöhlen- und Rachenbereich betroffen ist. In diesem Zusammenhang bilden Kopf-Hals-Tumoren in Mundhöhle und Rachen bei Männern die fünfthäufigste Krebserkrankung überhaupt. Überwiegend tritt der Kopf-Hals-Tumor nach dem sechzigsten Lebensjahr auf; die Häufigkeit von Kopf-Hals-Tumoren bei jüngeren Menschen steigt jedoch kontinuierlich an, wofür unter anderem der mitunter schon frühe Konsum von Nikotin und Alkohol verantwortlich gemacht wird. In speziellen Regionen der Welt sind Tumoren im Bereich von Kopf und Hals aufgrund verbreiteter Gewohnheiten, die zu den Risikofaktoren zählen, wie zum Beispiel der Konsum von Kautabak, besonders häufig.

Bösartige Tumoren der Nasenhaupt- und Nasennebenhöhlen machen zudem mehr als 10 % sämtlicher bösartiger Neubildungen des Kopf-Hals-Bereichs aus. Oftmals ist die Kieferhöhle zumindest mitbetroffen. Frühe Symptome können eine typischerweise einseitige Behinderung der Nasenluftpassage und sinusitische Beschwerden oder auch wiederholtes einseitiges Nasenbluten sein. Im weiteren Verlauf treten mitunter Schwellungen von Wange, Mundvorhof, Augenlidern und Stirn auf.

Was die Prognose von Krebserkrankungen des Kopf-Hals-Bereichs anbelangt, so ist diese im Frühstadium relativ gut, während sich die Heilungschancen bei entsprechendem Fortschritt der Erkrankung bzw. in Abhängigkeit von dem zugrundeliegenden Tumorstadium nachhaltig verschlechtern.

Die meisten bösartigen Tumoren im Kopf-Hals-Bereich werden erst in einem fortgeschrittenen Stadium entdeckt, was zu einer erheblichen Verschlechterung der Prognose führt. Die Therapie kann, abhängig von Tumorort, -art und -stadium, unterschiedlich bzw. differenziert ausfallen. Wie bei vielen anderen Krebserkrankungen auch, können die herkömmlichen Therapieformen, nämlich Chirurgie, Einsatz von Onkoziden (wie z.B. in Form einer Chemotherapie) und Strahlentherapie, und zwar auch in deren Kombination, zum Einsatz kommen. Bei Tumoren der Mundhöhle und des oberen Rachenraums ist ein chirurgischer Eingriff, verbunden mit einer Tumorresektion, die primäre Behandlungsmethode, welche im Allgemeinen und in Abhängigkeit vom Tumorstadium jedoch durch eine Bestrahlungs- und gegebenenfalls einer Chemotherapie ergänzt wird.

Obwohl hinsichtlich der im Stand der Technik bekannten therapeutischen Ansätze mitunter Fortschritte in Wirksamkeit und Verträglichkeit in Zusammenhang mit der Behandlung von Tumor- bzw. Karzinomerkrankungen des Kopf-Hals-Bereichs im Allgemeinen und Kopf-Hals-Plattenepithel-Karzinomen (HNSCC) im Speziellen gemacht wurden, sind die Mortalitätsraten insbesondere auch aufgrund der lokalen Tumorinvasion, der Entwicklung von Metastasen und dem Versagen von Chemo- und Strahlentherapien nach wie vor hoch, und dies bei hohen Nebenwirkungen bzw. Unverträglichkeiten der im Stand der Technik bekannten Therapieformen. Insbesondere haben sich die hohen Mortalitätsraten während der letzten drei Jahrzehnte nicht wesentlich geändert.

Auch von daher besteht im Stand der Technik ein hoher Bedarf, neue und innovative Therapieansätze zur Behandlung von Tumorerkrankungen bzw. Karzinomerkrankungen des Kopf-Hals-Bereichs bereitzustellen, zumal die im Stand der Technik bekannten therapeutischen Ansätze insbesondere auf Basis chirurgischer Eingriffe, Chemotherapie sowie Strahlentherapie mitunter mit starken Nebenwirkungen verbunden sind.

So betrifft die FR 2 956 817 A eine Zusammensetzung, welche mindestens einen Bestandteil von essentiellen Ölen der Pflanzen *Oregano compactum* oder *Aniba rosaeodora* umfasst, für eine therapeutische Verwendung in der Prävention oder der Behandlung von Keratosen im Transformationsstadium, präkanzerösen (prämaligne) oder kanzerösen Keratinozyten oder von aus der Transformation einer Keratose stammenden Karzinomen.

Weiterhin betrifft die wissenschaftliche Publikation gemäß T. Nishra et al., "Composition and in vitro cytotoxic activities of essential oil of Hedychium spicatum from different geographical regions of western Himalaya by principal components analysis", Natural Product Research, Band 30, Nr. 10, 21. Juli 2015, Seiten 1224 bis 1227 Zusammensetzungen und *In*-*vitro*-Untersuchungen zur zytotoxischen Aktivität ätherischer Öle von *Hedychium spicatum* aus verschiedenen geographischen Regionen des westlichen Himalaya-Gebirges mittels Analyse der Hauptkomponenten.

Schließlich fokussiert die WO 2014/141038 A2 auf eine Verabreichung von Wnt-Inhibitoren zu Therapiezwecken und beschreibt in diesem Zuge Verfahren zur Beobachtung einer differentiellen Genexpression von Biomarkern zur Bestimmung des Ansprechens eines Patienten auf einen Wnt-Inhibitor.

Vor diesem Hintergrund liegt daher der vorliegenden Erfindung die Aufgabe zugrunde, einen (wirk-)effizienten und mit verringerten Nebenwirkungen verbundenen bzw. mit einer verbesserten Verträglichkeit einhergehenden therapeutischen Ansatz zur Behandlung von insbesondere malignen Tumorerkrankungen bzw. Karzinomerkrankungen des Kopf-Hals-Bereichs bereitzustellen, wobei die zuvor geschilderten, in Zusammenhang mit dem Stand der Technik auftretenden Nachteile wenigstens teilweise vermieden oder aber wenigstens zumindest teilweise abgeschwächt werden sollen.

Insbesondere liegt der vorliegenden Erfindung die Aufgabe zugrunde, eine Zusammensetzung zur Verwendung bei der prophylaktischen bzw. therapeutischen Behandlung von Tumorerkrankungen bzw. Karzinomerkrankungen des Kopf-Hals-Bereichs, insbesondere von Kopf-Hals-Plattenepithel-Karzinomen (HNSCC), bereitzustellen, welche bei guter Verträglichkeit bzw. geringen Nebenwirkungen eine hohe therapeutische Wirkeffizienz aufweist und welche sich darüber hinaus auch zur kombinierten Verabreichung bzw. zur Kombination mit den im Stand der Technik bekannten Therapieansätzen, wie Chemotherapie oder dergleichen, eignet, um hierdurch insbesondere die Wirksamkeit der im Stand der Technik bekannten Therapieansätze zu verbessern.

Die der vorliegenden Erfindung zugrundeliegende Aufgabe besteht daher insbesondere in der Bereitstellung eines neuartigen Therapiekonzepts für die prophylaktische bzw. therapeutische Behandlung von Tumorerkrankungen bzw. Karzinomerkrankungen des Kopf-Hals-Bereichs, wobei ein solches Therapiekonzept gegenüber herkömmlichen Therapiemethoden mit weniger Nebenwirkungen und/oder Unannehmlichkeiten für die betroffenen Patienten verbunden sein und/oder eine mindestens vergleichbare, bevorzugt sogar verbesserte Wirkeffizienz bereitstellen soll, insbesondere auch in Kombination mit weiteren Behandlungsmaßnahmen.

Zur Lösung der zuvor geschilderten Aufgabenstellung schlägt die vorliegende Erfindung - gemäß einem ersten Aspekt der vorliegenden Erfindung - eine Zusammensetzung, insbesondere eine pharmazeutische Zusammensetzung, zur Verwendung bei der prophylaktischen bzw. therapeutischen Behandlung von insbesondere malignen Tumorerkrankungen bzw. Karzinomerkrankungen des Kopf-Hals-Bereichs nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem **zweiten** Aspekt der vorliegenden Erfindung - gleichermaßen ein Arzneimittel zur prophylaktischen bzw. therapeutischen Behandlung von malignen Tumorerkrankungen bzw. Karzinomerkrankungen des Kopf-Hals-Bereichs gemäß dem diesbezüglich unabhängigen und das Arzneimittel nach der Erfindung betreffenden Patentanspruch.

Zudem betrifft die vorliegende Erfindung - gemäß einem **dritten** Aspekt der vorliegenden Erfindung - die erfindungsgemäße Wirkstoffkombination, insbesondere in Form eines Kit (Kit-of-parts), gemäß dem diesbezüglich unabhängigen und die Wirkstoffkombination als solche betreffenden Patentanspruch.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung sämtlicher Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass der Rahmen der vorliegenden Erfindung verlassen wird.

Zudem gilt, dass sämtliche im Folgenden genannten Werte- bzw. Parameterangaben oder dergleichen grundsätzlich mit genormten bzw. standardisierten oder explizit angegeben Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr im Detail erläutert.

Gegenstand der vorliegenden Erfindung - gemäß einem **ersten** Aspekt der vorliegenden Erfindung - ist somit eine Zusammensetzung, insbesondere pharmazeutische Zusammensetzung, zur Verwendung bei der prophylaktischen und/oder therapeutischen Behandlung von malignen Tumorerkrankungen und/oder Karzinomerkrankungen des Kopf-Hals-Bereichs,
wobei die Tumorerkrankungen und/oder Karzinomerkrankungen des Kopf-Hals-Bereichs ausgewählt sind aus Kopf-Hals-Plattenepithel-Karzinomen (HNSCC),
wobei die Zusammensetzung als alleinigen Wirkstoff 1,8-Cineol enthält,
wobei das Cineol eine Reinheit von mindestens 95 Gew.-%, bezogen auf das Cineol, aufweist und wobei das Cineol frei von anderen Terpenen ist.

Erfindungsgemäß handelt es sich bei der zugrundeliegenden Erkrankung um solche Tumorerkrankungen bzw. Karzinomerkrankungen des Kopf-Hals-Bereichs, welche in Zusammenhang mit den sogenannten Wnt/β-Catenin-Signalweg stehen bzw. welche insbesondere hinsichtlich der Tumorinduktion bzw. -progression vom Wnt/β-Catenin-Signalweg reguliert bzw. beeinflusst werden, wie nachfolgend noch im Detail angeführt.

Die vorliegende Erfindung ist mit zahlreichen Vorteilen und Besonderheiten verbunden, welche nachfolgend in nichtbeschränkender Weise diskutiert sind und welche als Indiz für die Patentfähigkeit zu werten sind.

Im Rahmen der vorliegenden Erfindung kann erstmals ein neuartiges Therapiekonzept für die prophylaktische bzw. therapeutische Behandlung von Kopf-Hals-Plattenepithel-Karzinomen (HNSCC) bereitgestellt werden, mit welchem die zuvor geschilderten Nachteile des Standes der Technik zumindest weitgehend vermieden oder aber wenigstens abgeschwächt werden können. Insbesondere ist das erfindungsgemäße Therapiekonzept gegenüber herkömmlichen Therapiemethoden mit weniger Nebenwirkungen und/oder Unannehmlichkeiten für die betroffenen Patienten verbunden, wobei im Rahmen der vorliegenden Erfindung eine hohe Wirkeffizienz bereitgestellt wird. Insbesondere eignet sich das erfindungsgemäße Therapiekonzept auch zur gezielten Co-Medikation mit bekannten therapeutischen Ansätzen, wobei hierdurch die (Gesamt-)Wirksamkeit weiterführend gesteigert bzw. eine Dosisreduktion der weiterhin verabreichten Wirksubstanzen, wie Onkoziden (z.B. in Form von Chemotherapeutika) oder dergleichen, ermöglicht werden kann, insbesondere einhergehend mit einer Verringerung bzw. Minimierung von Nebenwirkungen.

Wie die Anmelderin überraschenderweise herausgefunden hat, lassen sich mit dem Wirkstoff Cineol, insbesondere 1,8-Cineol, bei der Behandlung von Tumorerkrankungen des Kopf-Hals-Bereichs, insbesondere von Kopf-Hals-Plattenepithel-Karzinomen (HNSCC), sehr gute Ergebnisse erzielen, wobei Cineol als natürlicher Wirkstoff nicht die mit den im Stand der Technik eingesetzten Wirksubstanzen, wie Chemotherapeutika, einhergehenden, mitunter schwerwiegenden Nebenwirkungen aufweist. Dabei kann das Cineol als Monotherapeutikum oder aber auch insbesondere bei der Behandlung schwerwiegender Fälle im Rahmen einer Co-Medikation eingesetzt werden, wobei durch die gemeinsame bzw. kombinierte Verwendung von Cineol die (Gesamt-)Wirkung verstärkt wird bzw. die Dosis der in Kombination verabreichten Wirksubstanzen mitunter reduziert werden kann (und dies bei zumindest gleicher Wirkeffizienz).

Dabei ist auch zu berücksichtigen, dass der Wirkstoff Cineol im Rahmen der vorliegenden Erfindung topisch (z. B. intranasal und/oder systemisch (z. B. peroral)) verabreicht werden kann, wobei hier insbesondere das lokale Wirkprofil von Cineol zum Tragen kommt. So kann bei topischer Anwendung das Cineol unmittelbar auf die Mund- und/oder Nasenschleimhäute aufgebracht werden, beispielsweise im Fall von Tumorerkrankungen der Nasenschleimhaut, wohingegen bei systemischer (z. B. peroraler) Anwendung das sozusagen ganzheitliche Wirkprofil von Cineol im Vordergrund steht.

Zudem lassen sich mit dem erfindungsgemäßen Konzept insbesondere auch rezidivierende Formen von Tumorerkrankungen des Kopf-Hals-Bereichs, insbesondere von Kopf-Hals-Plattenepithel-Karzinomen (HNSCC), behandeln. So kann insbesondere bei der Behandlung von postoperativen Zuständen die Rezidivrate verringert werden.

Wie nachfolgend gleichermaßen noch ausgeführt wird, lassen sich mit dem erfindungsgemäßen Therapiekonzept auch in Zusammenhang mit dem Wnt-Signalweg, insbesondere Wnt/β-Catenin-Signaltransduktionsweg, in Zusammenhang stehende Formen von Tumorerkrankungen des Kopf-Hals-Bereichs, insbesondere von Kopf-Hals-Plattenepithel-Karzinomen (HNSCC), behandeln, welche durch herkömmliche Therapiekonzepte des Standes der Technik bislang nicht immer in wirksamer Weise zufriedenstellend bzw. nur unter Inkaufnahme starker Nebenwirkungen therapiert werden konnten.

Insgesamt stellt daher die vorliegende Erfindung ein effizientes und flexibel einsetzbares Therapiekonzept bereit, welches auf einen natürlichen Wirkstoff (nämlich Cineol, insbesondere 1,8-Cineol) zurückgreift, mit welchem keine nennenswerten unerwünschten Nebenwirkungen verbunden sind. Insbesondere kann das erfindungsgemäße Therapiekonzept in effizienter und flexibler Weise auch mit herkömmlichen Therapiekonzepten kombiniert werden; so kann beispielsweise das erfindungsgemäße Therapiekonzept in Kombination mit Chemotherapeutika oder dergleichen sowie nach einer chirurgischen Entfernung von Tumoren bzw. Karzinomen insbesondere in Kombination bzw. in Ergänzung mit herkömmlichen Wirkstoffen zur Verhinderung der Rezidivbildung eingesetzt werden, insbesondere mit der Maßgabe, deren Wirkweise zu verstärken bzw. deren Dosis zu reduzieren (und damit auch deren Nebenwirkungsprofil).

Im Rahmen der vorliegenden Erfindung sind die Tumorerkrankungen und/oder Karzinomerkrankungen des Kopf-Hals-Bereichs ausgewählt aus Kopf-Hals-Plattenepithel-Karzinomen (HNSCC). Mit anderen Worten handelt es sich erfindungsgemäß bei der zugrundeliegenden Erkrankung um ein Kopf-Hals-Plattenepithel-Karzinom (HNSCC).

Darüber hinaus können die Tumorerkrankungen bzw. Karzinomerkrankungen des Kopf-Hals-Bereichs ausgewählt sein aus der Gruppe von Tumorerkrankungen und/oder Karzinomerkrankungen der Mundhöhle, des Mundrachens, der inneren Nase, der Nasennebenhöhlen, des Nasenrachens, des Kehlkopfes, des Hypopharynx, des Ohres, der Gesichtshaut, der Halshaut und der Speicheldrüsen. Gemäß einer erfindungsgemäß bevorzugten Ausführungsform sind die Tumorerkrankungen bzw. Karzinomerkrankungen des Kopf-Hals-Bereichs ausgewählt aus der Gruppe von Tumorerkrankungen bzw. Karzinomerkrankungen der Mundhöhle, des Mundrachens, der inneren Nase, der Nasennebenhöhlen und des Nasenrachens.

Was die zugrundeliegenden Tumorerkrankungen bzw. Karzinomerkrankungen weiterhin anbelangt, so können diese ausgewählt sein aus der Gruppe von Mundhöhlenkarzinomen, Oropharynxkarzinomen, Nasenhöhlenkarzinomen, Nasopharynxkarzinomen, Hypopharynxkarzinomen, Larynxkarzinomen und Trachealkarzinomen. Insbesondere können die Tumorerkrankungen bzw. Karzinomerkrankungen ausgewählt sein aus der Gruppe von Mundhöhlenkarzinomen, Oropharynxkarzinomen, Nasenhöhlenkarzinomen und Nasopharynxkarzinomen.

Erfindungsgemäß handelt es sich auch bei den zuvor genannten Erkrankungen um Formen des Kopf-Hals-Plattenepithel-Karzinoms (HNSCC).

Was die vorliegende Erfindung weiterhin anbelangt, so kann die erfindungsgemäße Zusammensetzung bzw. das Cineol zur prophylaktischen bzw. therapeutischen Behandlung von mit dem Wnt-Signalweg, insbesondere Wnt/β-Catenin-Signaltransduktionsweg, in Zusammenhang stehenden Formen von Tumorerkrankungen bzw. Karzinomerkrankungen des Kopf-Hals-Bereichs, vorzugsweise zur prophylaktischen bzw. therapeutischen Behandlung von über den Wnt-Signalweg, insbesondere Wnt/β-Catenin-Signaltransduktionsweg, induzierten und/oder proliferierten Formen von Tumorerkrankungen und/oder Karzinomerkrankungen des Kopf-Hals-Bereichs, eingesetzt werden.

Erfindungsgemäß kann die Zusammensetzung bzw. das Cineol gleichermaßen zur prophylaktischen bzw. therapeutischen Behandlung von mit dem Wnt-Signalweg, insbesondere Wnt/β-Catenin-Signaltransduktionsweg, in Zusammenhang stehenden malignen Tumor- bzw. Karzinomerkrankungen des Kopf-Hals-Bereichs, vorzugsweise Kopf-Hals-Karzinomen, bevorzugt Plattenepithelkarzinomen, bzw. vorzugsweise zur prophylaktischen und/oder therapeutischen Behandlung von über den Wnt-Signalweg, insbesondere Wnt/β-Catenin-Signaltransduktionsweg, induzierten und/oder proliferierten malignen Tumor- bzw. Karzinomerkrankungen des Kopf-Hals-Bereichs, vorzugsweise Kopf-Hals-Karzinomen, bevorzugt Plattenepithelkarzinomen, eingesetzt werden.

Derartige Formen bzw. Zustände von Kopf-Hals-Karzinomen, bevorzugt in Form von Plattenepithelkarzinomen, können bislang mit herkömmlichen Therapiekonzepten nicht bzw. nicht immer zufriedenstellend behandelt oder therapiert werden.

Der sogenannte Wnt-Signalweg, insbesondere Wnt/β-Catenin-Signaltransduktionsweg, ist einer von vielen Signaltransduktionswegen, mittels welchem Zellen auf äußere Signale reagieren können. Dieser Signalweg ist nach seinem Liganden "Wnt" benannt, einem Signalprotein, welches als lokaler Mediator eine wichtige Funktion bei der Entwicklung verschiedener tierischer Zellen einnimmt.

Das Akronym "Wnt" selbst ist historisch begründet und setzt sich aus den Bestandteilen "Wg" einerseits und "Int-1" andererseits zusammen: Der Akronymteil "W" entstammt dem Kürzel "Wg", welches wiederum für das englische Wort "wingless" (flügellos) steht und denjenigen Wachstumsfaktor der Wnt-Familie bezeichnet, welcher als erster entdeckt wurde (wenn das Wg-Gen mutiert ist, entsteht bei der Taufliege *Drosophila melanogaster* eine flügellose Variante.). Das Akronymteil "nt" stammt von dem Int-Gen, welches bei der Maus die Entwicklung von Brustkrebs fördert bzw. triggert, wenn es aktiviert ist. Insgesamt wurden inzwischen 19 verschiedene Wnt-Gene identifiziert.

An der Signaltransduktion des Wnt-Signalweges sind - ohne sich auf diese Theorie beschränken zu wollen - zahlreiche Proteine beteiligt. Er ist essentiell für die normale Embryonalentwicklung (Embryogenese) und wird auch bei bestimmten Krebsformen beim Menschen beobachtet. Nach derzeitigem Wissensstand bindet das Wnt-Signalprotein an den Rezeptor "*Frizzled*" (zusammen mit dem Co-Rezeptor "LRP" [*low-density lipoprotein receptor-related protein]),* welcher das Protein *"Dishevelled* (DVL)" aktiviert, welches wiederum inhibierend auf einen Proteinkomplex (bestehend aus GSK-3 [einer Proteinkinase], dem Tumorsuppressorprotein APC und dem Protein Axin-1) einwirkt, welcher normalerweise β-Catenin abbaut. Da nun die Degradation von β-Catenin inhibiert ist, sammelt sich dieses im Cytoplasma und im Zellkern an. Im Zellkern bildet das β-Catenin mit TCF/LEF einen Protein-Komplex und aktiviert auf diese Weise spezifische Zielgene. Das APC-Gen ist ein Tumorsuppressorgen, welches zuerst bei einem bestimmten Typ von Darmkrebs mutiert gefunden wurde, jedoch kann dieses Gen auch bei anderen Krebsarten mutieren; das APC-Gen kodiert für das APC-Protein, welches im Wnt-Signalweg an der Degradation von β-Catenin beteiligt ist.

Der Wnt-Signalweg ist bei vielen Vorgängen im Rahmen der Embryonalentwicklung (Embryogenese) von Bedeutung, wie z. B. bei der Ausbildung der Körperachse und der Bildung von Organanlagen. In adulten Zellen hingegen ist er zumeist inaktiv, in Tumorzellen kann er aber wieder aktiviert vorliegen.

In Zellen, in welchen der Wnt-Signalweg inaktiv ist, liegt β-Catenin - gleichermaßen ohne sich auf diese Theorie beschränken zu wollen - in einem Komplex gebunden vor, welcher dazu führt, dass β-Catenin ständig abgebaut wird; auf diese Weise wird β-Catenin daran gehindert, die Transkription bestimmter Gene zu aktivieren. Dieser Komplex, welcher auch als *Destruction-Komplex* bezeichnet, besteht aus verschiedenen Proteinen, wobei zu den wichtigsten Axin, die Proteinkinase GSK-3 und das Tumorsuppressorprotein APC gehören. Wenn β-Catenin in diesem Komplex vorliegt, kann es von β-TrCP gebunden und ubiquitiniert werden; diese Ubiquitinierung führt dazu, dass β-Catenin im Proteasom abgebaut wird. Wenn aber "Wnt" an seinen Rezeptor "*Frizzled*" und den Co-Rezeptor "LRP" bindet, wird das Protein "DVL" aktiviert, welches den *Destruction-Komplex* inhibiert; β-Catenin wird freigesetzt und kann nicht mehr abgebaut werden, und es akkumuliert und gelangt auch in den Zellkern, wo es mit anderen Proteinen zusammen an einen Transkriptionsfaktor bindet, welcher auf diese Weise aktiviert wird, was wiederum in der Transkription verschiedener Gene (z. B. Cyclin D1 und MYC) resultiert.

In diesem Zusammenhang hat die Anmelderin in völlig überraschender Weise gefunden, dass die relevanten Formen bzw. Ausbildungen der der vorliegenden Erfindung zugrundeliegenden Erkrankung in Zusammenhang mit dem in Rede stehenden Wnt-Signalweg, insbesondere Wnt/β-Catenin-Signaltransduktionsweg, stehen, und zwar insbesondere insofern, als die relevanten Formen der Erkrankungen in Zusammenhang mit einer Aktivierung des in Rede stehenden Signalwegs stehen und hierdurch induziert werden können, und zwar insbesondere dahingehend, dass die Erkrankungen in ihrem Verlauf in für den Patienten nachteiliger Weise beschleunigt bzw. begünstigt werden.

Insbesondere hat die Anmelderin dabei völlig überraschend gefunden, dass das im Rahmen der vorliegenden Erfindung als Wirksubstanz eingesetzte Cineol, insbesondere 1,8-Cineol, imstande ist, den Wnt-Signalweg, insbesondere Wnt/β-Catenin-Signaltransduktionsweg, zu beeinflussen und insbesondere zu inhibieren, was mit einer verringerten Zellproliferation bzw. Tumorpromotion hinsichtlich der in Rede stehenden relevanten Formen der Erkrankungen und somit mit einer günstigeren Verlaufsprognose für den betroffenen Patienten einhergeht.

Mit anderen Worten wurde im Rahmen der vorliegenden Erfindung erstmalig und in völlig überraschender Weise gefunden, dass Cineol, insbesondere 1,8-Cineol, als Inhibitor bzw. Suppressor des Wnt-Signalwegs, insbesondere Wnt/β-Catenin-Signaltransduktionsweg, fungiert, was zu der entsprechenden pharmakologischen Wirkeffizienz hinsichtlich der zugrundeliegenden Tumorerkrankungen bzw. Karzinomerkrankungen des Kopf-Hals-Bereichs, insbesondere in Form von Kopf-Hals-Plattenepithel-Karzinomen (HNSCC), führt.

In diesem Zusammenhang haben seitens der Anmelderin durchgeführte Untersuchungen gezeigt, dass Cineol, insbesondere 1,8-Cineol, zu einer Inhibition des Wnt/β-Catenin-Signalwegs, führt, wobei - ohne sich auf diese Theorie beschränken zu wollen - insbesondere in Gegenwart von Cineol eine Abnahme von aktivem β-Catenin, bei welchem es sich um ein zentrales transkriptionelles Aktivatorprotein des zugrundeliegenden Signalwegs handelt, vorliegt. In diesem Zusammenhang führt - gleichermaßen ohne sich auf diese Theorie beschränken zu wollen - Cineol bzw. 1,8-Cineol zu einem geringeren Level bzw. Gehalt an phosphorylierter GSK-3-Proteinkinase, wie nachfolgend definiert, was zu einer verstärkten Aktivität von GSK-3 und somit zu einer verstärkten Degradation von β-Catenin führt, einhergehend mit einer Inhibierung bzw. Inaktivierung des zugrundeliegenden Wnt/β-Catenin-Signaltransduktionswegs.

Bei GSK-3 handelt es sich - ohne sich auf diese Theorie beschränken zu wollenum eine konstitutiv aktive Proteinkinase, welche insbesondere durch die Phosphorylierung an Serin-9 (GSK-3α) oder Serin-21 (GSK-3β) inhibiert wird. Die Verringerung der Phosphorylierung von GSK-3 an diesen Stellen, wie durch Cineol hervorgerufen, führt im Rahmen der dem Wnt/β-Catenin-Signalweg zugrundeliegenden Signalkaskade somit zu einer Aktivierung von GSK-3 als konstitutiv aktive Proteinkinase, was wiederum zu einer verstärkten Phosphorylierung und damit Inaktivierung bzw. Degradation von β-Catenin führt.

In der Folge führt die durch Cineol induzierte bzw. hervorgerufene Inaktivierung des Wnt/β-Catenin-Signalwegs in Bezug auf die zu behandelnden Erkrankungen (d.h. insbesondere maligne Tumor- bzw. Karzinomerkrankungen des Kopf-Hals-Bereichs, vorzugsweise Kopf-Hals-Karzinome, bevorzugt Plattenepithelkarzinome) zu einer verringerten Tumorpromotion bzw. zu einer verringerten Zellproliferation und somit zu einer Tumorsuppression bzw. Abschwächung oder gar Heilung der Erkrankung bzw. zu einer Verbesserung des Gesundheitszustand. Hierzu kann auch auf die nachfolgenden Ausführungen verwiesen werden.

Darüber hinaus führt die erfindungsgemäße Verwendung von Cineol - gleichermaßen ohne sich auf diese Theorie beschränken zu wollen - auch zu einer verringerten endolysomalen Lokalisation von GSK-3, was dessen verringerte Inhibition infolge der Behandlung mit Cineol unterstützt und was wiederum mit einer weiteren Inaktivierung von β-Catenin einhergeht.

Das erfindungsgemäß eingesetzte Cineol führt somit im Hinblick auf den zugrundeliegenden Wnt/β-Catenin-Signalweg sozusagen in mehrfacher Weise zu einer entsprechenden Inhibition der zugrundeliegenden Signalkaskade, einhergehend mit der zuvor angeführten Wirkeffizienz in Bezug auf die zu behandelnden Tumor- bzw. Krebserkrankungen.

Die Anmelderin hat gleichermaßen völlig überraschend gefunden, dass im Hinblick auf die zugrundeliegenden Erkrankungen - gleichermaßen ohne sich auf diese Theorie beschränken zu wollen - die Anwendung von Cineol zu einer Verringerung der Aktivität bzw. Verringerung der Expression mindestens eines insbesondere mit dem Wnt/β-Catenin-Signalweg in Zusammenhang stehenden Gens, vorzugsweise Wnt-Gens (synonym auch als WNT-Gen bezeichnet), insbesondere des Gens Wnt11 (synonym auch als WNT11-Gen bezeichnet), führt.

In diesem Zusammenhang fungiert das Cineol insbesondere als Regulator, vorzugsweise Inhibitor und/oder Suppressor, für die in Rede stehenden Gene bzw. Genprodukte, z.B. in Form der entsprechenden Proteine, insbesondere als Regulator für mindestens ein Wnt-Gen und/oder Wnt-Protein (synonym auch als WNT-Protein bezeichnet), insbesondere des Wnt11-Gens und/oder des Wnt11-Proteins (synonym auch als WNT11-Protein bezeichnet). Im Allgemeinen kann die Regulation, insbesondere Inhibition und/oder Suppression, somit auf Gen-, Transkriptions-, Translations- und/oder Proteinebene erfolgen.

In diesem Zusammenhang hat die Anmelderin auch gefunden, dass eine verringerte Aktivität bzw. eine verringerter Expression von Wnt11 insbesondere zu einer verringerten zellulären Progression im Hinblick auf die zugrundeliegenden Erkrankungen (d.h. maligne Tumor- bzw. Karzinomerkrankungen des Kopf-Hals-Bereichs, vorzugsweise Kopf-Hals-Karzinome, bevorzugt Plattenepithelkarzinome) führt.

Zusammenfassend wurde erfindungsgemäß somit erstmals gefunden, dass Cineol, insbesondere 1,8-Cineol, in der Lage ist, die Aktivität des Wnt/β-Catenin-Signaltransduktionsweges mit pharmakologischer Relevanz für die zugrundeliegenden Tumorerkrankungen bzw. Karzinomerkrankungen des Kopf-Hals-Bereichs, insbesondere Kopf-Hals-Plattenepithel-Karzinom (HNSCC), zu inhibieren, was zu einer verringerten zellulären Progression der Tumor- bzw. Karzinomerkrankungen führt.

Erfindungsgemäß wird somit erstmalig ein neuer pharmakologischer Effekt des natürlichen Wirkstoffs Cineol aufgezeigt. Der für Cineol aufgefundene Wirkeffekt mit der einhergehenden Wirkeffizienz für die zugrundeliegenden Erkrankungen werden durch die seitens der Anmelderin durchgeführten Untersuchungen in eindrucksvoller Weise belegt, wie nachfolgend noch im Rahmen der Ausführungsbeispiele ausführlich referiert.

Was die vorliegende Erfindung weiterhin anbelangt, so kann die Zusammensetzung nach der Erfindung bzw. das Cineol auch zur prophylaktischen bzw. therapeutischen Behandlung von postoperativen Zuständen eingesetzt werden. Beispielsweise kann die Zusammensetzung bzw. das Cineol nach erfolgter Tumorresektion bzw. operativer Tumorentfernung eingesetzt werden, insbesondere vor dem Hintergrund der Vermeidung bzw. Verringerung eines Tumorrezidivs. Erfindungsgemäß reduziert bzw. minimiert der Einsatz von Cineol, insbesondere 1,8-Cineol, im Rahmen des erfindungsgemäßen Therapiekonzepts die Bildung von Rezidiven in wirksamer Weise.

Somit kann die Zusammensetzung nach der Erfindung zur prophylaktischen bzw. therapeutischen Behandlung von Tumorerkrankungen bzw. Karzinomerkrankungen des Kopf-Hals-Bereichs nach vorangehender chirurgischer Entfernung von Tumoren bzw. nach vorangehender Tumorresektion eingesetzt werden, insbesondere wie zuvor angeführt.

Im Rahmen der vorliegenden Erfindung ist es vorgesehen, dass die Zusammensetzung nach der Erfindung das Cineol als Reinstoff, d.h. frei von anderen Terpenen, enthält. In diesem Zusammenhang ist es erfindungsgemäß vorgesehen, dass das Cineol mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, eingesetzt wird. Hierdurch wird eine besonders hohe Wirkeffizienz bei gleichzeitig nochmals verbesserter Verträglichkeit erreicht.

Erfindungsgemäß ist es somit vorgesehen, dass Cineol als Reinstoff vorliegt bzw. eingesetzt wird. Erfindungsgemäß ist das Cineol frei von anderen Terpenen bzw. enthält keine anderen Terpene.

Insbesondere kann das Cineol als 1,8-Cineol vorliegen. Denn auf der Basis der Verwendung eines speziellen Wirkstoffs in Form von 1,8-Cineol kann im Rahmen der vorliegenden Erfindung eine besonders gute Wirkeffizienz bzw. pharmakologische Wirksamkeit in Bezug auf die erfindungsgemäße Zusammensetzung erreicht bzw. eingestellt werden.

Was den im Rahmen der vorliegenden Erfindung zum Einsatz kommenden natürlichen Wirkstoff Cineol, insbesondere 1,8-Cineol anbelangt, so ist diesbezüglich Folgendes auszuführen:
1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist. Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum. Darüber hinaus ist 1,8-Cineol beispielsweise in Niaouli-, Juniperus-, Piper-, Cannabis-, Kajeput-, Salbeiöl, Myrtenöl und anderen ätherischen Ölen enthalten. Technisches 1,8-Cineol, welches im Allgemeinen 99,6- bis 99,8-%ig ist, wird im Allgemeinen durch fraktionierte Destillation von Eukalyptusöl gewonnen. 1,8-Cineol wird im Stand der Technik insbesondere als Expektorans bei Bronchialkatarrhen und anderen Atemwegserkrankungen vorwiegend in der Veterinärmedizin, aber darüber hinaus auch als Aromastoff in der Parfümindustrie angewendet. Darüber hinaus wird 1,8-Cineol in der Zahnmedizin bei der Revision von Wurzelfüllungen verwendet.

In pharmakologischer Hinsicht wirkt 1,8-Cineol in den oberen und unteren Atemwegen, insbesondere in der Lunge und den Nasennebenhöhlen, schleimlösend und bakterizid. Außerdem hemmt es bestimmte Neurotransmitter, welche z. B. für die Verengung von Bronchien verantwortlich sind (bei chronischobstruktiven Lungenerkrankungen und Asthma bronchiale kann daher durch Gabe von reinem 1,8-Cineol die Lungenfunktion verbessert werden.). Aufgrund seiner kortikosteroidartigen Wirkung kann Cineol als Substitut zu oder in Co-Medikation mit Kortikosteroiden angewendet werden. 1,8-Cineol kann -wie nachfolgend noch ausgeführt - grundsätzlich sowohl topisch, z. B. (intra)nasal und/oder inhalativ, oder aber systemisch, insbesondere peroral (z. B. in Form von Kapseln), angewendet werden.

Als Wirkstoff besitzt 1,8-Cineol also schleimlösende und entzündungshemmende Wirkungen. Bei systemischer Anwendung wird 1,8-Cineol leicht resorbiert und gelangt über die Blutbahn in den Atmungsorganen zur Wirkung. 1,8-Cineol kann auf diese Weise beispielsweise entzündliche Sekrete sowie zähen Schleim in den Luftwegen verflüssigen und entzündlichen Prozessen in den Atemwegen entgegenwirken. Im Bereich der oberen Luftwege schwinden die Behinderung der Nasenatmung bei Schnupfen und die Benommenheit des Kopfes.

Für weitergehende Einzelheiten zu dem Wirkstoff 1,8-Cineol kann beispielsweise verwiesen werden auf Römpp Chemielexikon, Georg Thieme Verlag, Stuttgart/New York, 10. Auflage, Band 1, 1996, Seite 752, Stichwort: "Cineol", sowie die dort referierte Literatur.

Durch die erfindungsgemäß bevorzugte Verwendung von Cineol als Reinsubstanz, insbesondere in Form von 1,8-Cineol, wird ein definiertes bzw. steuerbares Wirkprofil gewährleistet. Insbesondere werden infolge der Abwesenheit weiterer Terpene unerwünschte oder unvorhersehbare Wechsel- und Nebenwirkungen vermieden.

Erfindungsgemäß kann es gleichermaßen vorgesehen sein, dass das Cineol in liposomenumgebener bzw. liposomal verpackter Form vorliegt. Auf diese Weise wird insbesondere eine verbesserte Löslichkeit bzw. Emulgierbarkeit des Cineols bei gleichzeitig guter Stabilisierung des Cineols in der Zusammensetzung und bei guter Bioverfügbarkeit erreicht.

Erfindungsgemäß ist es vorgesehen, dass die Zusammensetzung nach der Erfindung das Cineol, insbesondere in Form von 1,8-Cineol, als alleinigen Wirkstoff, insbesondere als alleinigen pharmazeutischen Wirkstoff, enthält. Gleichermaßen wird im Rahmen der vorliegenden Erfindung das Cineol, insbesondere in Form von 1,8-Cineol, als alleiniger Wirkstoff, insbesondere als alleiniger pharmazeutischer Wirkstoff, eingesetzt. Hierdurch wird ein besonders definiertes Wirkprofil gewährleistet, wobei zudem mitunter unerwünschte Wechselwirkungen mit weiteren Wirksubstanzen vermieden werden, was auch der Verträglichkeit der erfindungsgemäßen Zusammensetzung zugute kommt.

Die Zusammensetzung kann das Cineol in wirksamen, insbesondere pharmazeutisch wirksamen Mengen enthalten. Hierdurch werden insbesondere die erfindungsgemäß aufgeführten Wirkeffekte gewährleistet.

In diesem Zusammenhang kann die Menge an Cineol in der cineolhaltigen Zusammensetzung nach der vorliegenden Erfindung, insbesondere in Abhängigkeit von der Therapieform (z. B. topisch oder systemisch) und der Schwere und Eigenart der zu behandelnden Erkrankung, in weiten Bereichen variieren bzw. eingestellt werden. Insbesondere kann es vorgesehen sein, dass die Zusammensetzung das Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält. Auf diese Weise werden eine gute Wirkeffizienz einerseits und eine gute Verträglichkeit andererseits gewährleistet. Dennoch kann es einzelfallbedingt oder anwendungsbezogen erforderlich sein, von den vorgenannten Werten abzuweichen, ohne dass der Rahmen der vorliegenden Erfindung verlassen ist. Dies liegt im Ermessen des Fachmanns.

Insbesondere kann es erfindungsgemäß vorgesehen sein, wenn die erfindungsgemäße Zusammensetzung das Cineol, bezogen auf die Zusammensetzung, in relativen Mengen von mindestens 0,0001 Gew.-%, insbesondere von mindestens 0,001 Gew.-%, vorzugsweise von mindestens 0,005 Gew.-%, bevorzugt von mindestens 0,01 Gew.-%, besonders bevorzugt von mindestens 0,05 Gew.-%, ganz besonders bevorzugt von mindestens 0,1 Gew.-%, enthält.

Weiterhin kann es erfindungsgemäß insbesondere vorgesehen sein, wenn die erfindungsgemäße Zusammensetzung das Cineol, bezogen auf die Zusammensetzung, in relativen Mengen von höchstens 80 Gew.-%, insbesondere von höchstens 75 Gew.-%, vorzugsweise von höchstens 70 Gew.-%, bevorzugt von höchstens 60 Gew.-%, besonders bevorzugt von höchstens 55 Gew.-%, ganz besonders bevorzugt von höchstens 50 Gew.-%, enthält.

Gemäß einer weiteren Ausführungsform der vorliegenden Erfindung kann es zudem vorgesehen sein, dass die Zusammensetzung das Cineol zusammen mit mindestens einem physiologisch unbedenklichen Träger (Exzipienten) enthält bzw. dass das Cineol zusammen mit mindestens einem physiologisch unbedenklichen Träger (Exzipienten) eingesetzt wird.

In diesem Zusammenhang kann es erfindungsgemäß vorgesehen sein, dass der Träger (Exzipient) mit 1,8-Cineol mischbar bzw. hierin löslich ist bzw. dass der Träger (Exzipient) bei 20 °C und bei Atmosphärendruck im flüssigen oder festen Aggregatzustand vorliegt.

Gemäß einer erfindungsgemäß bevorzugten Ausführungsform kann der Träger bzw. Exzipient, insbesondere im Hinblick auf eine systemische Applikationsform, ausgewählt sein aus der Gruppe von fetten Ölen, bevorzugt Triglyceriden, besonders bevorzugt mittelkettigen Triglyceriden (*Medium Chain Triglycerides,* MCT), ganz besonders bevorzugt Triglyceriden mit C₆-C₁₂-Fettsäureestern.

Der Begriff der Öle, wie er erfindungsgemäß für die vorstehenden Träger bzw. Exzipienten verwendet wird, ist eine Sammelbezeichnung für Flüssigkeiten, welche sich nicht mit Wasser mischen lassen. Der Begriff der fetten Öle, wie er in diesem Zusammenhang erfindungsgemäß verwendet wird, bezeichnet insbesondere Fette, d.h. Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den so genannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe "*tri*" auf drei Acyl-Säurereste, die mit Glycerin verestert sind, verweist.

Speziell mittelkettige Triglyceride, wie sie erfindungsgemäß bevorzugt als Träger bzw. Exzipient für den Wirkstoff 1,8-Cineol zum Einsatz kommen, sind insbesondere halbsynthetische neutrale Glycerinester von gesättigten, im Allgemeinen unverzweigten Monocarbonsäuren mittlerer Kettenlänge (d.h. C₆-C₁₂). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, das aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur. 6., Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*"*).

Im Allgemeinen kann es erfindungsgemäß vorgesehen sein, dass die Zusammensetzung nach der Erfindung topisch oder systemisch appliziert wird bzw. dass die Zusammensetzung zur topischen oder systemischen Applikation hergerichtet ist.

Insbesondere kann das Cineol topisch oder systemisch appliziert werden bzw. kann das Cineol zur topischen oder systemischen Applikation hergerichtet sein.

Insbesondere kann die Zusammensetzung bzw. das Cineol topisch, insbesondere intranasal oder inhalativ, appliziert werden. Insbesondere kann die Zusammensetzung bzw. das Cineol zur topischen, insbesondere intranasalen oder inhalativen Applikation hergerichtet sein. In diesem Zusammenhang kann die Zusammensetzung bzw. das Cineol insbesondere in Form einer intranasal zu verabreichenden Darreichungsform appliziert werden. Gleichermaßen kann die Zusammensetzung bzw. das Cineol in einer intranasal zu verabreichenden Darreichungsform hergerichtet sein. Insbesondere kann die Zusammensetzung bzw. das Cineol in diesem Zusammenhang in Form von Nasensprays, Nasentropfen, Nasengelen, Nasensalben, Nasenspülungen, Nasencremes, Inhalaten oder dergleichen, bevorzugt Nasensprays, appliziert werden. Gleichermaßen kann die Zusammensetzung in diesem Zusammenhang bzw. das Cineol in diesem Zusammenhang in Form von Nasensprays, Nasentropfen, Nasengelen, Nasensalben, Nasenspülungen, Nasencremes, Inhalaten oder dergleichen, bevorzugt Nasensprays, hergerichtet sein. Eine intranasale Applikation kommt beispielsweise bei entsprechenden Tumorerkrankungen der inneren Nase, der Nasennebenhöhle bzw. des Nasenrachens in Betracht, wobei die in Rede stehende Applikationsform nicht auf die zuvor genannten Indikationen beschränkt ist. Der Fachmann ist jederzeit in der Lage, die entsprechende Applikationsform vor dem Hintergrund der zugrundeliegenden Krebserkrankungen speziell auszuwählen bzw. abzustimmen.

Gemäß einer weiteren Ausführungsform bzw. Variante der vorliegenden Erfindung kann es zudem vorgesehen sein, dass die Zusammensetzung bzw. das Cineol systemisch, insbesondere peroral oder parenteral (z. B. intravenös, intraarteriell, intramuskulär, subkutan etc.; beispielsweise in Form einer parenteralen Darreichungsform, wie Injektion, Infusion etc.), vorzugsweise peroral, appliziert wird. Insbesondere kann die Zusammensetzung bzw. das Cineol zur systemischen, insbesondere peroralen oder parenteralen, vorzugsweise peroralen Applikation hergerichtet sein.

In diesem Zusammenhang kann die Zusammensetzung bzw. das Cineol in Form einer peroral oder parenteral, vorzugsweise peroral, zu verabreichenden Darreichungsform appliziert werden. Gleichermaßen kann die Zusammensetzung bzw. das Cineol in diesem Zusammenhang in einer peroral oder parenteral, vorzugsweise peroral, zu verabreichenden Darreichungsform hergerichtet sein.

Zudem kann die Zusammensetzung bzw. das Cineol als magensaftresistente, aber dünndarmlösliche systemische Darreichungsform, bevorzugt Kapsel, Dragee, Pille, Tablette oder dergleichen, appliziert werden. In diesem Zusammenhang kann die Zusammensetzung bzw. das Cineol gleichermaßen als magensaftresistente, aber dünndarmlösliche systemische Darreichungsform, bevorzugt Kapsel, Dragee, Pille, Tablette oder dergleichen, hergerichtet sein.

Eine perorale Applikation kommt beispielsweise im Hinblick auf die Behandlung von Tumorerkrankungen der Mundhöhle, des Mundrachens, des Kehlkopfes, des Hypopharynx und dergleichen in Betracht. Auch diesbezüglich ist der Fachmann jederzeit in der Lage, die entsprechende Applikationsform vor dem Hintergrund der zugrundeliegenden Indikation konkret auszuwählen bzw. diesbezüglich anzupassen.

Was zudem die systemische Darreichung bzw. Applikation der Zusammensetzung nach der Erfindung anbelangt, so kann diesbezüglich sozusagen eine ganzheitliche bzw. systemische Wirksamkeit des Cineols hervorgerufen werden, was gleichermaßen von therapeutischem Vorteil sein kann. Im Rahmen der systemischen Applikation unter Verwendung magensaftresistenter, dünndarmlöslicher Darreichungsformen kann insbesondere erreicht werden, dass der Wirkstoff in Form von Cineol bei systemischer Applikation erst am eigentlichen Resorptionsort, d.h. dem Dünndarm, freigesetzt wird. Die Bereitstellung entsprechender Darreichungs- bzw. Applikationsformen mit magensaftresistenter, dünndarmlöslicher Beschichtung sind dem Fachmann als solche wohlbekannt, so dass es diesbezüglich keiner weiterführenden Ausführungen bedarf.

Insbesondere in Abhängigkeit von der Art der Darreichung (d.h. systemisch und/oder topisch) kann die Tagesdosis des applizierten Cineols in weiten Bereichen variieren. Was die erfindungsgemäße Zusammensetzung somit weiterhin anbelangt, so kann das Cineol mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht werden. Insbesondere kann die Zusammensetzung bzw. das Cineol zur Verabreichung mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, hergerichtet sein.

Insbesondere kann das Cineol systemisch mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, verabreicht werden. In diesem Zusammenhang kann die Zusammensetzung bzw. das Cineol zur Verabreichung mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, hergerichtet sein.

Zudem kann das Cineol topisch mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, verabreicht werden. In diesem Zusammenhang kann die Zusammensetzung bzw. das Cineol zur Verabreichung mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, hergerichtet sein.

Hinsichtlich der erfindungsgemäß auszuwählenden Tagesdosen ist der Fachmann jederzeit in der Lage, die diesbezüglich konkreten Werte insbesondere im Hinblick auf die zugrundeliegende Indikation bzw. Verabreichung auszuwählen und weiterführend abzustimmen.

Erfindungsgemäß kann es zudem vorgesehen sein, dass die Zusammensetzung außerdem mindestens einen weiteren Inhaltsstoff, insbesondere mindestens einen Hilfsstoff bzw. ein Additiv, enthält. Insbesondere kann das Cineol mit mindestens einem weiteren Inhaltsstoff, insbesondere mindestens einem Hilfsstoff bzw. Additiv, eingesetzt werden.

In diesem Zusammenhang kann der mindestens eine weitere Inhaltsstoff, insbesondere Hilfsstoff und/oder Additiv, ausgewählt sein aus der Gruppe von Verarbeitungshilfsstoffen, Stabilisatoren, Emulgatoren, Antioxidantien, Feuchthaltemitteln, Verdickungsmitteln, Desinfektionsmitteln, pH-Stellmitteln, pH-Puffersubstanzen, Konservierungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen bzw. ätherischen Ölen sowie deren Kombinationen. Was die vorliegende Erfindung darüber hinaus anbelangt, so kann es vorgesehen sein, dass die Zusammensetzung bzw. das Cineol in Ergänzung, bevorzugt in Kombination, mit Onkoziden, insbesondere ausgewählt aus der Gruppe von Antitumormitteln, Chemotherapeutika und Cytostatika, bzw. in Ergänzung, bevorzugt in Kombination, mit Strahlentherapie eingesetzt wird.

Gleichermaßen kann es erfindungsgemäß vorgesehen sein, dass die Zusammensetzung bzw. das Cineol als Ergänzungstherapeutikum (Co-Therapeutikum), bevorzugt als Kombinationstherapeutikum, mit Onkoziden, insbesondere ausgewählt aus der Gruppe von Antitumormitteln, Chemotherapeutika und Cytostatika, und/oder mit Strahlentherapie eingesetzt wird. Hierdurch kann die Wirksamkeit des Onkozids bzw. der Strahlentherapie verbessert werden bzw. können die entsprechenden Dosen reduziert werden.

Weiterhin kann es erfindungsgemäß vorgesehen sein, dass die Zusammensetzung bzw. das Cineol, insbesondere in Co-Therapie oder in Kombination mit Onkoziden, insbesondere ausgewählt aus der Gruppe von Antitumormitteln, Chemotherapeutika und Cytostatika, und/oder mit Strahlentherapie zur Reduktion des Bedarfs oder zum Ersatz von Onkoziden, insbesondere ausgewählt aus der Gruppe von Antitumormitteln, Chemotherapeutika und Cytostatika, und/oder von Strahlentherapie bei der prophylaktischen und/oder therapeutischen Behandlung von Tumorerkrankungen und/oder Karzinomerkrankungen des Kopf-Hals-Bereichs eingesetzt wird.

Erfindungsgemäß ist es gleichermaßen möglich, dass die Zusammensetzung bzw. das Cineol, insbesondere in Co-Therapie oder in Kombination mit Onkoziden, insbesondere ausgewählt aus der Gruppe von Antitumormitteln, Chemotherapeutika und Cytostatika, und/oder mit Strahlentherapie zur Wirkungssteigerung und/oder Dosisreduktion von Onkoziden, insbesondere ausgewählt aus der Gruppe von Antitumormitteln, Chemotherapeutika und Cytostatika, und/oder von Strahlentherapie bei der prophylaktischen und/oder therapeutischen Behandlung von Tumorerkrankungen und/oder Karzinomerkrankungen des Kopf-Hals-Bereichs eingesetzt wird.

Gemäß einer weiteren erfindungsgemäßen Ausführungsform kann es zudem vorgesehen sein, dass die Zusammensetzung bzw. das Cineol, insbesondere in Co-Therapie oder in Kombination mit Onkoziden, insbesondere ausgewählt aus der Gruppe von Antitumormitteln, Chemotherapeutika und Cytostatika, und/oder mit Strahlentherapie zur Verringerung oder Vermeidung von Nebenwirkungen von Onkoziden, insbesondere ausgewählt aus der Gruppe von Antitumormitteln, Chemotherapeutika und Cytostatika, und/oder von Strahlentherapie bei der prophylaktischen und/oder therapeutischen Behandlung von Tumorerkrankungen und/oder Karzinomerkrankungen des Kopf-Hals-Bereichs eingesetzt wird.

Erfindungsgemäß kann es zudem vorgesehen sein, dass die Zusammensetzung bzw. das Cineol nicht in Ergänzung, insbesondere nicht in Kombination, mit nichtsteroidalen Entzündungshemmern (NSAID) eingesetzt wird bzw. dass die Zusammensetzung und/oder das Cineol nicht als Ergänzungstherapeutikum (Co-Therapeutikum), insbesondere nicht als Kombinationstherapeutikum, mit nichtsteroidalen Entzündungshemmern (NSAID) eingesetzt wird. Auf diese Weise werden die unerwünschten Nebenwirkungen von NSAID in wirksamer Weise verhindert (zumal deren Verwendung im Rahmen des erfindungsgemäßen Therapiekonzepts nicht erforderlich ist).

Im Rahmen der vorliegenden Erfindung ist es somit vorgesehen, Cineol, insbesondere 1,8-Cineol, zur Verwendung bei der prophylaktischen bzw. therapeutischen Behandlung von malignen Tumorerkrankungen bzw. Karzinomerkrankungen des Kopf-Hals-Bereichs, insbesondere von Kopf-Hals-Karzinomen, bevorzugt Plattenepithelkarzinomen, einzusetzen. Wie zuvor angeführt, handelt es sich bei der zugrundeliegenden Erkrankung um Kopf-Hals-Plattenepithel-Karzinome (HNSCC).

Mit anderen Worten betrifft die vorliegende Erfindung die Verwendung einer Cineol, vorzugsweise 1,8-Cineol, als pharmazeutischen Wirkstoff enthaltenden Zusammensetzung, insbesondere pharmazeutischen Zusammensetzung, zur prophylaktischen bzw. therapeutischen Behandlung von malignen Tumorerkrankungen bzw. Karzinomerkrankungen des Kopf-Hals-Bereichs, insbesondere von Kopf-Hals-Karzinomen, bevorzugt Plattenepithelkarzinomen, bzw. zur Herstellung eines Arzneimittels zur prophylaktischen bzw. therapeutischen Behandlung von malignen Tumorerkrankungen bzw. Karzinomerkrankungen des Kopf-Hals-Bereichs, insbesondere von Kopf-Hals-Karzinomen, bevorzugt Plattenepithelkarzinomen.

Auch betrifft die vorliegende Erfindung die Verwendung von Cineol, insbesondere 1,8-Cineol, als pharmazeutischer Wirkstoff zur prophylaktischen bzw. therapeutischen Behandlung von malignen Tumorerkrankungen bzw. Karzinomerkrankungen des Kopf-Hals-Bereichs, insbesondere von Kopf-Hals-Karzinomen, bevorzugt Plattenepithelkarzinomen, bzw. zur Herstellung eines Arzneimittels zur prophylaktischen bzw. therapeutischen Behandlung von malignen Tumorerkrankungen bzw. Karzinomerkrankungen des Kopf-Hals-Bereichs, insbesondere von Kopf-Hals-Karzinomen, bevorzugt Plattenepithelkarzinomen.

Für weitergehende Einzelheiten zu diesem Erfindungsaspekt kann auf die nachfolgenden Ausführungen zu den übrigen Erfindungsaspekten verwiesen werden, welche in Bezug auf diesen Erfindungsaspekt entsprechend gelten.

Die vorliegende Erfindung betrifft weiterhin auch - gemäß einem **zweiten** Aspekt der vorliegenden Erfindung - Arzneimittel zur Verwendung bei der prophylaktischen und/oder therapeutischen Behandlung von malignen Tumorerkrankungen und/oder Karzinomerkrankungen des Kopf-Hals-Bereichs, wobei die Tumorerkrankungen und/oder Karzinomerkrankungen des Kopf-Hals-Bereichs ausgewählt sind aus Kopf-Hals-Plattenepithel-Karzinomen (HNSCC), wobei das Arzneimittel als alleinigen Wirkstoff 1,8-Cineol enthält, wobei das Cineol eine Reinheit von mindestens 95 Gew.-%, bezogen auf das Cineol, aufweist und wobei das Cineol frei von anderen Terpenen ist.

Für weitergehende Einzelheiten zu diesem Erfindungsaspekt kann auf die vorangehenden Ausführungen zu den übrigen Erfindungsaspekten verwiesen werden, welche in Bezug auf diesen Erfindungsaspekt entsprechend gelten.

Zudem betrifft die vorliegende Erfindung - gemäß einem **dritten** Aspekt der vorliegenden Erfindung - auch eine Wirkstoffkombination, insbesondere in Form eines Kit (Kit-of-parts), zur Verwendung bei der prophylaktischen und/oder therapeutischen Behandlung von malignen Tumorerkrankungen und/oder Karzinomerkrankungen des Kopf-Hals-Bereichs, wobei die Tumorerkrankungen und/oder Karzinomerkrankungen des Kopf-Hals-Bereichs ausgewählt sind aus Kopf-Hals-Plattenepithel-Karzinomen (HNSCC),
wobei die Wirkstoffkombination als räumlich getrennte, aber funktional zusammenhängende Komponenten
(a) 1,8-Cineol in Form einer Cineol als alleinigen Wirkstoff enthaltenden, topisch oder systemisch zu applizierenden pharmazeutischen Zusammensetzung, wobei das Cineol eine Reinheit von mindestens 95 Gew.-%, bezogen auf das Cineol, aufweist und wobei das Cineol frei von anderen Terpenen ist, einerseits und
(b) mindestens ein Onkozid, ausgewählt aus der Gruppe von Antitumormitteln, Chemotherapeutika und Cytostatika, in Form einer das Onkozid enthaltenden, topisch oder systemisch zu applizierenden pharmazeutischen Zusammensetzung,
umfasst.

Für weitergehende Einzelheiten zu diesem Erfindungsaspekt kann auf die vorangehenden Ausführungen zu den übrigen Erfindungsaspekten verwiesen werden, welche in Bezug auf diesen Erfindungsaspekt entsprechend gelten.

Im Ergebnis ist es im Rahmen der vorliegenden Erfindung möglich, Cineol, insbesondere 1,8-Cineol, zur Regulation, vorzugsweise Inhibition und/oder Suppression, des Wnt-Signalwegs, insbesondere Wnt/β-Catenin-Signaltransduktionswegs, vorzugsweise zur Regulation und/oder Verringerung der Phosphorylierung der GSK-3-Proteinkinase, insbesondere an Serin-9 (GSK-3α) und/oder Serin-21 (GSK-3β), im Rahmen des Wnt-Signalwegs bzw. zur Aktivierung der GSK-3-Proteinkinase und/oder zur Deaktivierung und/oder Phosphorylierung von β-Catenin im Rahmen des Wnt-Signalwegs, bei der prophylaktischen und/oder therapeutischen Behandlung von malignen Tumorerkrankungen und/oder Karzinomerkrankungen des Kopf-Hals-Bereichs, insbesondere von Kopf-Hals-Karzinomen, bevorzugt Plattenepithelkarzinomen, zu verwenden.

Gleichermaßen ist es erfindungsgemäß vorgesehen, Cineol, insbesondere 1,8-Cineol, bei der prophylaktischen und/oder therapeutischen Behandlung von mit dem Wnt-Signalweg, insbesondere Wnt/β-Catenin-Signaltransduktionsweg, in Zusammenhang stehenden und/oder hierdurch induzierten und/oder begünstigten Erkrankungen, vorzugsweise von malignen Tumorerkrankungen und/oder Karzinomerkrankungen des Kopf-Hals-Bereichs, insbesondere von Kopf-Hals-Karzinomen, bevorzugt Plattenepithelkarzinomen, zu verwenden.

Dabei wird das Cineol insbesondere als Regulator, vorzugsweise Inhibitor und/oder Suppressor, des Wnt-Signalwegs, insbesondere Wnt/β-Catenin-Signaltransduktionswegs, vorzugsweise als Regulator und/oder Verringerer der Phosphorylierung der GSK-3-Proteinkinase, insbesondere an Serin-9 (GSK-3α) und/oder Serin-21 (GSK-3β), im Rahmen des Wnt-Signalwegs bzw. zur Aktivierung der GSK-3-Proteinkinase und/oder als Deaktivator von β-Catenin im Rahmen des Wnt-Signalwegs, eingesetzt.

Weiterhin kann das Cineol auch als Regulator, vorzugsweise Inhibitor und/oder Suppressor, mindestens eines Wnt-Gens und/oder Wnt-Proteins, insbesondere des Wnt11-Gens und/oder Wnt11-Proteins, eingesetzt werden.

Weitere vorteilhafte Eigenschaften, Aspekte und Merkmale der vorliegenden Erfindung ergeben sich aus der folgenden Beschreibung von in den Figuren dargestellten Ausführungsbeispielen, wie sie auch nachfolgend noch im Detail angeführt sind. Es zeigt:

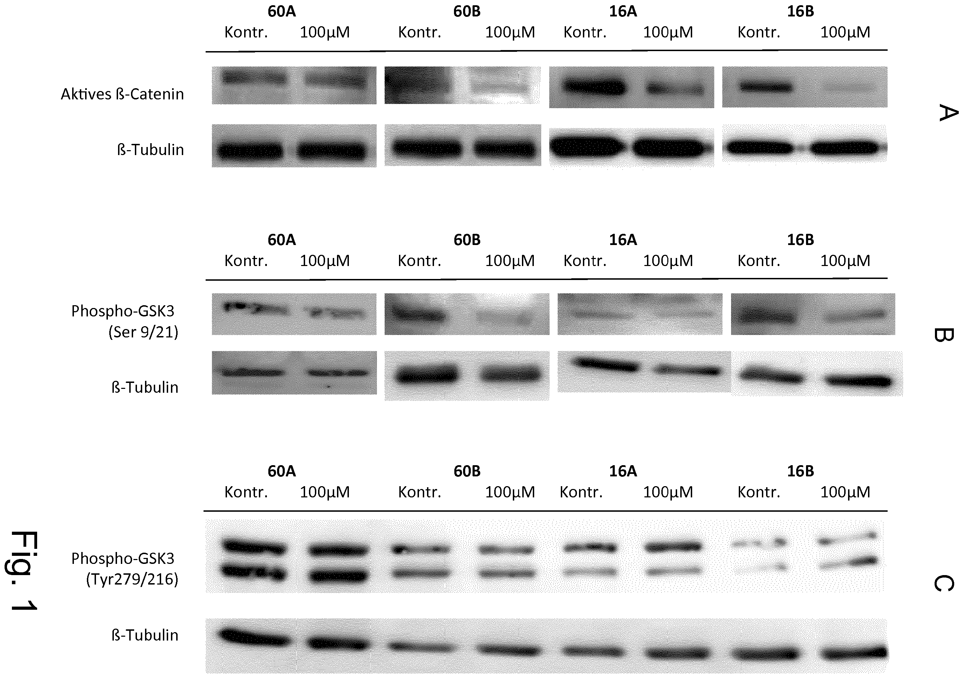

- Fig. 1: die Expression von aktivem β-Catenin und P-GSK3 (Serin-9/-21) als Reaktion auf Cineol; hierzu werden Western Blot-Analysen an Proteinen aus ganzen Zellen eingesetzt, um den Effekt einer 24 h-Behandlung mit 100 µM 1,8-Cineol auf den Proteinexpressionsgrad von aktivem β-Catenin (Fig. 1A), Phospho-GSK-3 (Serin-9/-21) (Fig. 1B) und Phospho-GSK-3 (Tyrosin-279/-216) (Fig. 1C) in permanenten HNSCC-Zelllinien 60A/B und 16A/B zu zeigen; als Beladungskontrolle wird β-Tubulin eingesetzt;

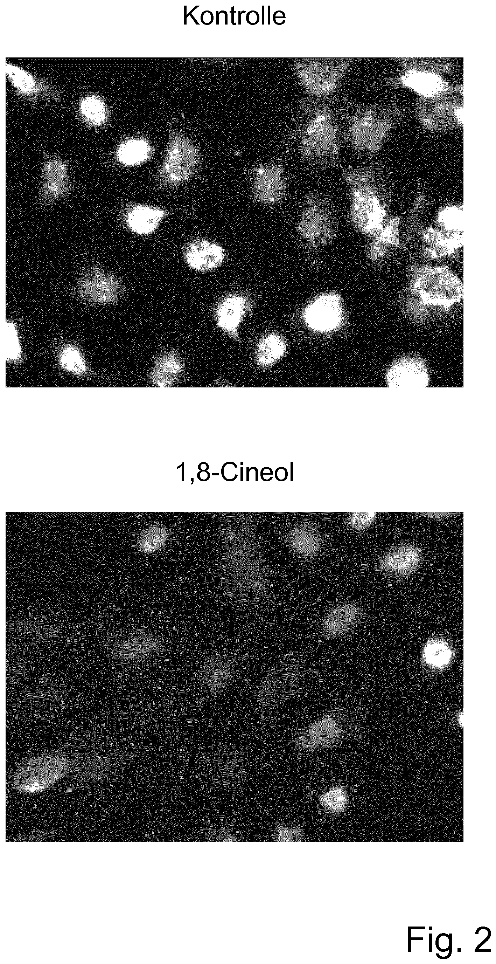

- Fig. 2: die subzelluläre Lokalisation von P-GSK-3 (Serin-9/-21) als Reaktion auf Cineol; immunohistochemische Untersuchungen zeigen einen Rückgang der endolysosomalen Lokalisation von Phospho-GSK-3 (Serin-9/-21) als Reaktion auf 100 µM 1,8-Cineol im Vergleich zum Kontrollmedium; Zellkerne sind unter Einsatz von DAPI gefärbt;

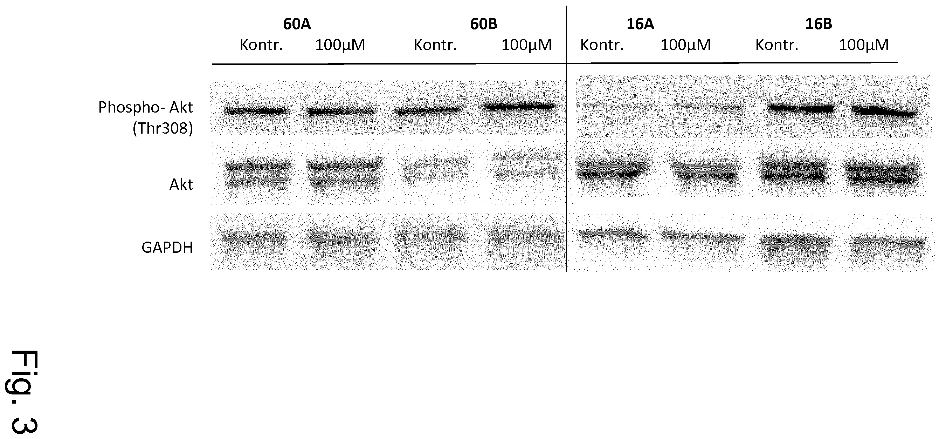

- Fig. 3: die Expression von Akt und P-Akt (Threonin-308) als Reaktion auf 1,8-Cineol; Western Blot-Analysen von Proteinen aus ganzen Zellen werden eingesetzt, um den Effekt von 100 µM 1,8-Cineol auf das Proteinexpressionslevel von Akt und P-Akt (Threonin-308) in permanenten HNSCC-Zelllinien 60A/B und 16A/B zu zeigen; als Beladungskontrolle wird β-Tubulin eingesetzt;

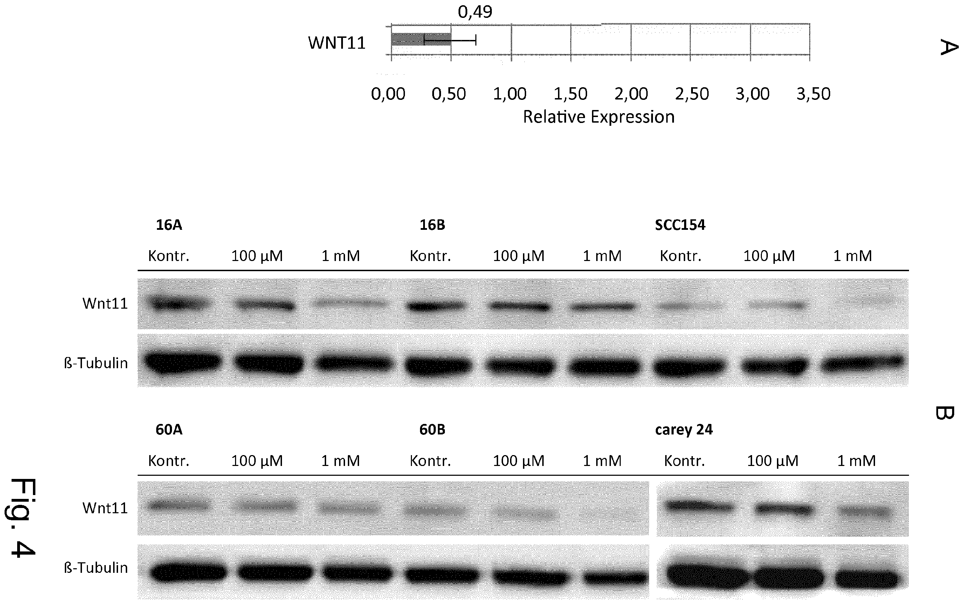

- Fig. 4: die Expression von Wnt11 als Reaktion auf Cineol; die Expressionslevel von Wnt11 werden in Bezug auf die mRNA (Fig. 4A) sowie auf Proteinebene (Fig. 4B) unter Einsatz des RT²-PCR-Profiler und Western Blot-Experimenten bestimmt; Western Blot-Analysen von Proteinen aus ganzen Zellen werden eingesetzt, um den Effekt von 1,8-Cineol auf das Proteinexpressionslevel in permanenten HNSCC-Zelllinien 60A/B, 16AB/B, SCC154 und Carey24 zu zeigen; als Beladungskontrolle wird β-Tubulin eingesetzt;

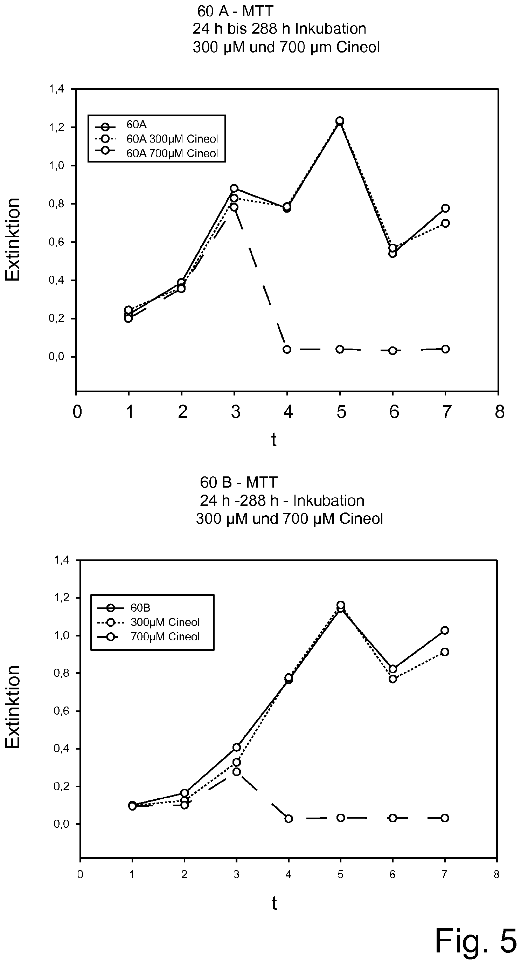

- Fig. 5: die dosisabhängige Verringerung der Lebensfähigkeit von HNSCC-Zelllinien durch 1,8-Cineol; um zu bestimmen, ob die Behandlung mit 1,8-Cineol zu einer Inhibition der zellulären Lebensfähigkeit von HNSCC führt, werden quantitative kolorimetrische 3-(4,5-Dimethylthiazol-2-yl)-2,5-diphenyltetrazoliumbromid-basierte Zelltests (MTT-Tests) durchgeführt; als Reaktion auf eine Inkubation mit 700 µM 1,8-Cineol kann eine stark verringerte zelluläre Lebensfähigkeit von analysierten HNSCC-Zellen beobachtet werden;

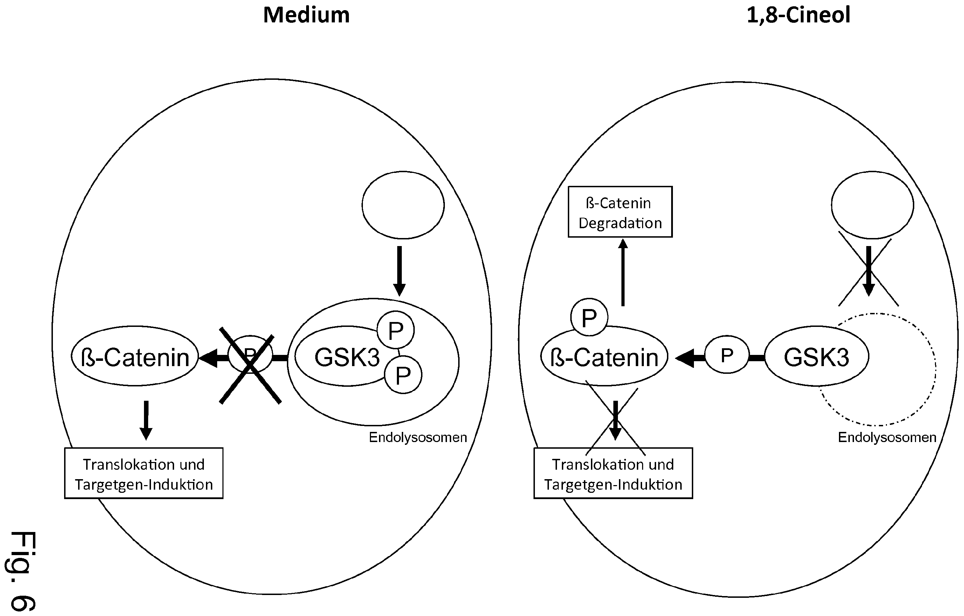

- Fig. 6: ein Modell der Wnt/β-Catenin-Inhibition als Reaktion auf 1,8-Cineol; im Rahmen der vorliegenden Erfindung hat die Anmelderin erstmals gezeigt, dass 1,8-Cineol als Inhibitor für die Wnt/β-Catenin-Aktivität fungiert und einen starken Rückgang der zellulären Progression von Tumorzellen bewirkt; die Daten der Anmelderin zeigen, dass 1,8-Cineol die inhibitorische Phosphorylierung und endolysosomale Lokalisierung von GSK3, insbesondere GSK3-β, bei welchem es sich um den zentralen Regulator der β-Catenin-Aktivität handelt, beeinflusst.

Für weitergehende Einzelheiten zu der in den Figurendarstellungen dargestellten Ausführungsformen und Ausführungsbeispielen kann zur Vermeidung unnötiger Wiederholungen auf die obigen Ausführungen sowie auf die nachfolgenden ergänzenden Ausführungen zu den Ausführungsbeispielen verwiesen werden, welche in Bezug auf die Figurendarstellung entsprechend gelten.

Zusammenfassend ist in Bezug auf die vorliegende Erfindung hervorzuheben, dass es sich bei Kopf-Hals-Plattenepithel-Karzinomen (HNSCC) um eine der häufigsten üblichen soliden Neoplasien weltweit handelt. Deren Auftreten steht insbesondere in Zusammenhang mit Tabakrauch-Exposition, dem Konsum von Alkohol sowie mit Virusinfektionen, wie Infektionen mit HPV. Obwohl große Fortschritte in Zusammenhang mit der Behandlung von HNSCC gemacht wurden, sind die Mortalitätsraten aufgrund der lokalen Tumorinvasion, der Entwicklung von Metastasen und dem Versagen von Chemo- und Strahlentherapien nach wie vor hoch.

Die vorliegende Erfindung weist auf eine wichtige Rolle des Wnt/β-Catenin-Signalwegs in der humanen Tumorgenese insbesondere des Kopf-Hals-Plattenepithel-Karzinoms (HNSCC) hin. Auf dieser Basis können spezielle Proteine dieses Signalwegs als wirksames Ziel für einen neuen therapeutischen Ansatz unter Verwendung von Cineol als Wirksubstanz dienen.

Insbesondere wird im Rahmen der vorliegenden Erfindung - ohne sich auf diese Theorie beschränken zu wollen - eine aktive Beteiligung des Wnt/β-Regulator-Proteins Glycogen-Synthase-Kinase 3 (GSK-3) an den in Rede stehenden Krebserkrankungen aufgezeigt. So kann eine pathologische bzw. übermäßige Inhibition bzw. Phosphorylierung von GSK-3 an bestimmten Phosphorylierungsstellen die Tumorbildung durch die Aktivierung von β-Catenin anregen. Dementsprechend kann GSK-3 als Tumorsuppressor in seiner durch Cineol begünstigten, an bestimmten Phosphorylierungsstellen nicht phosphorylierten Form fungieren. Insbesondere kann GSK-3 als ein kritischer Regulator der Aktivität des Kernfaktors κB (NFκB) angenommen werden. Die regulierte inhibitorische Phosphorylierung insbesondere an Serin-9, wie zuvor angeführt, kann als Ursache für die Aktivierung von Wnt angenommen werden, wobei diese in epithelialen Kanzerosen hochreguliert ist. Die Wnt-Signalwege sind im Allgemeinen für die Bestimmung der Gewebeentwicklung, die Differenzierung und Embryogenese, die Tumorprogression und die Invasivität von Bedeutung.

Insgesamt sind bislang mindestens 19 Wnt-Homologe im Menschen bekannt, von denen einige als potentielle Signalmoleküle für verschiedene Typen von Krebs identifiziert wurden. Vorliegend kann insbesondere gezeigt werden, dass höhere Level bzw. Mengen an aktivem β-Catenin mit einer schlechten Prognose bei HNSCC korrelieren. Eine Inhibition der Wnt/β-Catenin-Kaskade kann in diesem Zusammenhang als Strategie bzw. pharmakologisches Target bzw. Ziel zur Behandlung von HNSCC herangezogen werden.

Die im Rahmen der vorliegenden Erfindung aufgefundenen Ergebnisse zeigen, dass Cineol, insbesondere 1,8-Cineol, bei welchem es sich insbesondere um den aktiven Wirkstoff des klinisch zugelassenen Arzneimittels Soledum® handelt, als ein Inhibitor der Wnt/β-Catenin-Aktivität fungiert und zu einer verringerten zellulären Progression bei HNSCC führt. Insbesondere kann im Rahmen der vorliegenden Erfindung der Einfluss von Cineol, insbesondere 1,8-Cineol, auf die Regulation des Wnt/β-Catenin-Signalwegs in verschiedenen permanenten Zelllinien von HNSCC aufgezeigt werden.
Die Ergebnisse zeigen erstmals, dass Cineol, insbesondere 1,8-Cineol, in der Lage ist, die Wnt/β-Catenin-Aktivität in HNSCC zu inhibieren und zu einem verringerten Expressionslevel von Wnt11 und zu einer verringerten zellulären Progression zu führen. Somit zeigen die erfindungsgemäß aufgeführten Ergebnisse eine neue Aktivität bzw. einen neuen Therapieansatz (Therapiekonzept) von Cineol, insbesondere 1,8-Cineol, was zu neuen therapeutischen Ansätzen für diesen natürlichen Wirkstoff führt, wie erfindungsgemäß definiert.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.
Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Allgemeine Ausführungen:

Die vorliegende Erfindung weist auf eine wichtige Rolle des Wnt/β-Catenin-Signalwegs in der humanen Tumorgenese von Kopf-Hals-Tumorerkrankungen, insbesondere in Form des Kopf-Hals-Plattenepithel-Karzinoms (HNSCC), hin. Insbesondere zeigt die vorliegende Erfindung eine aktive Beteiligung des Wnt/β-Regulator-Proteins Glycogen-Synthase-Kinase 3 (GSK-3) an den in Rede stehenden Krebserkrankungen auf, und zwar insbesondere im Rahmen einer Tumorsuppression bzw. als Tumorsuppressor in seiner durch Cineol begünstigten und in Bezug auf bestimmte Phosphorylierungsstellen nicht phosphorylierten Form.

Die erfindungsgemäß aufgefundenen Ergebnisse zeigen, dass Cineol, insbesondere 1,8-Cineol, als ein Inhibitor der Wnt/β-Catenin-Aktivität fungiert und zu einer verringerten zellulären Progression bei HNSCC führt. Erfindungsgemäß kann der Einfluss von Cineol, insbesondere 1,8-Cineol, auf die Regulation des Wnt/β-Catenin-Signalwegs in verschiedenen permanenten Zelllinien von HNSCC aufgezeigt werden.

Die Ergebnisse zeigen erstmals, dass Cineol, insbesondere 1,8-Cineol, in der Lage ist, die Wnt/β-Catenin-Aktivität in HNSCC zu inhibieren und zu einem verringerten Expressionslevel von Wnt11 und zu einer verringerten zellulären Progression führt. Somit zeigen die erfindungsgemäß aufgeführten Ergebnisse eine neue Aktivität (Mechanismus) von Cineol, insbesondere 1,8-Cineol, was zu neuen therapeutischen Ansätzen bzw. Therapiekonzepten führt, wie erfindungsgemäß definiert und zuvor beschrieben.

### 2. Material und Methoden

### Zellen und Bedingungen für Zellkulturen

Die im Rahmen der vorliegenden Erfindung untersuchten adhärenten humanen Kopf-Hals-Plattenepithel-Karzinom-Zelllinien (UT-SCC16A, UT-SCC-16B, UT-SCC-60A und UT-SCC-60B) werden in "Dulbecco's Modifiziertem Eagle's Medium" (DMEM, 4,5 g/l D-Glucose und L-Glutamin), welches mit 10 % fötalem Rinderserum (FBS) (beide Life Technologies, Carlsbad, CA, USA), 1 % Natriumpyruvat (PAN-Biotech, Aidenbach, Deutschland) und 1 % Antibiotika (10.000 U/ml Penicillin, 10.000 µg/ml Streptomycin) (Biochrom, Berlin, Deutschland) supplementiert ist, bei 37 °C unter 5 % CO₂ und 95 % LuftAtmosphäre gelagert.

### Antikörper

Anti-Phospho-GSK-3α/β-Antikörper (Serin-21/-9) (#9331) und Anti-GAPDH-Antikörper (#2118) werden über Cell Signaling Technology (Danvers, MA, USA) bezogen. Beide Antikörper werden in einer 10: 1.000-Verdünnung in TBS mit 3 % BSA (PAA Laboraties, Pasching, Österreich) verwendet. Anti-Phospho-GSK-3a/β-Antikörper (Y279/Y216) (#ab6847) werden von Abcam (Cambridge, UK) erhalten und in einer 1 : 1.000-Verdünnung in TBS mit 3 % BSA verwendet. Der gegen aktives-β-Catenin gerichtete Antikörper (#05-665) wird über Merck Milipore (Billerica, MA, USA) bezogen und in einer Verdünnung von 1 : 2.000 in TBS und 3 % Milchpulver (Carl Roth, Karlsruhe, Deutschland) verwendet. Die speziesspezifischen sekundären Antikörper Anti-Maus IgG (ganzes Molekül)-Peroxidase (# A9044) und Anti-Kaninchen IgG (ganzes Molekül)-Peroxidase (# A0545) werden von Sigma-Aldrich (St. Louis, MO, USA) bezogen und in einer Verdünnung von 1 : 50.000-Verdünnung in TBS mit entweder 3 % BSA oder 3 % Trockenmilchpulver verwendet.

### Western Blot

Die Zellen werden zweimal in eiskaltem PBS gewaschen und anschließend in RIPA-Puffer (Cell Signaling Technology, Danvers, MA, USA) mit Aprotinin, Pepstatin A, Protease Inhibitor Cocktail und Phenylmethansulfonylfluorid (Sigma-Aldrich, St. Louis, MO, USA) für 1,5 Stunden lysiert. Durch Zentrifugation (13.000 rpm, 4 °C, 10 Minuten) werden Zelltrümmer entfernt. Die Proteinkonzentration wird mittels des Bradford-Tests mit einfach konzentriertem Quick StartTM Dye Reagent (Bio-Rad Laboratories, Hercules, CA, USA) bestimmt. Es wird vierfach konzentrierter SDS-Probenpuffer hinzugefügt, und die Proben werden für 5 Minuten bei 95 °C denaturiert. Äquivalente Proteinmengen (30 µg) werden mittels SDS-PAGE in einem 10 % Polyacrylamidgel aufgetrennt und mittels Immunoblot auf Nitrocellulosemembranen übertragen. Für die Proteinextraktion aus Geweben werden die Gewebe zunächst in 500 µl RIPA-Puffer mit Protease-Inhibitoren unter Einsatz des TissueLyser LT (Qiagen, Hilden, Deutschland) homogenisiert, anschließend inkubiert und wie zuvor beschrieben zentrifugiert.

Die Membranen werden mit TBS, enthaltend 3 % BSA oder 3 % Milchpulver, für 1 Stunde bei Raumtemperatur geblockt und anschließend mit dem primären Antikörper über Nacht bei 4 °C inkubiert. Nach einem Waschschritt mit TBS werden die Membranen mit dem speziesspezifischen sekundären Antikörper für 1 Stunde bei Raumtemperatur inkubiert. Die Proteinbanden werden unter Einsatz von Amersham™ ECL™ Primer Western Blotting Detection Reagent (GE Healthcare, Chalfont St Giles, Buckinghamshire, UK) und Fusion-FX7 sowie der korrespondierenden Software Fusion Bio-1D V12.14 (Vilber-Lourmat, Eberhardzell, Deutschland) nach ausgiebigem Waschen visualisiert. Die Nitrozellulosemembranen werden über Nacht bei 4 °C mit einem GAPDH-Antikörper inkubiert, um die Beladung jeder Spur zu bestimmen. Die Membranen werden nochmals gewaschen und mit dem sekundären Antikörper und ECL Detektionsreagenz inkubiert.

PCR-Test zur humanen epithelial-mesenchymalen Transition (EMT) Mit dem "Human Epithelial to Mesenchymal Transition (EMT) RT² Profiler™ PCR Array" wird die Expression von 84 Schlüsselgenen, welche ihre Expression während dieses Prozesses verändern oder die Expression von sich verändernden Genen regulieren, festgestellt. Der Test umfasst für Zelloberflächenrezeptoren, extrazelluläre Matrix und das Zytoskelett kodierende Gene, Gene, welche Zelladhäsion, Migration, Motilität und Morphogenese steuern; sowie Gene für Signaltransduktion und Transkriptionsfaktoren, welche die EMT sowie die damit assoziierten Prozesse bedingen.

### Immunohistochemie

Zur Detektion von E-Cadherin innerhalb der Tumorzellen wird eine immunohistochemische Methode eingesetzt. Die Zellen werden 24 Stunden vor der Behandlung mit 100 µM Cineol in 4-Well-Kammerobjektträgern (Sarstadt AG, Numbrecht, Deutschland) überführt. Nach der Inkubation wurde das Medium entfernt und die Zellen wurden zweimal in PBS gewaschen. Zur intrazellulären Färbung wurden die Zellen mit 0,1 % Triton-X-100 (Sigma-Aldrich Chemie GmbH, Steinheim) in PBS fixiert. Der spezifische primäre Antikörper Anti-Phospho-GSK-3α/β (Serin-21/-9) (#9331) (Cell Signaling, Danvers, MA, USA) wird mit einem Antikörper-Verdünnungspuffer, welcher Blocking-Puffer (DCS LabLine, DCS, Hamburg, Deutschland) enthält, auf 1 : 50 verdünnt und in einer luftbefeuchteten Kammer für 1 Stunde bei Raumtemperatur (RT) inkubiert. Nach Waschen mit PBS wird der zweite Antikörper Ziege-anti-Maus FITC (Jackson ImmunoResearch Laboratories, West Grove, USA) in einer Verdünnung von 1 : 100 für 45 Minuten inkubiert. Die Zellen werden dreimal mit PBS gespült. Die Zellkerne werden für 1 Minute unter Einsatz von DAPI (1 µg/ml, Roche Diagnostics, Mannheim, Deutschland) gefärbt. Die Proben werden dreimal für 5 Minuten mit PBS gespült und anschließend in Fluoromount G (Southern Biotechnologies Associates, Birmingham, Alabama, USA) eingebettet. Die Proben werden unter einem 200M-Mikroskop (Zeiss, Göttingen, Deutschland) mittels Fluoreszenzmikroskopie (AHF Analysentechnik AG, Tübingen, Deutschland) ausgewertet. Die Zellen wurden mithilfe der AxioCam MRm Rev.3 FireWire und der AxioVision Rel. 4.7 Software (Zeiss, Jena, Deutschland) fotografiert.

### 1,8-Cineol

Der Wirkstoff wird in Form von 1,8-Cineol aus Soledum® Kapseln eingesetzt (Klosterfrau Healthcare Group, Cassella-med GmbH & Co. KG, Köln, Deutschland). Die aus Eukalyptusöl isolierte Reinsubstanz, d. h. reines 1,8-Cineol mit einer Reinheit > 99 % (0,6 mg/µl bzw. 600 mg/ml 1,8-Cineol), wird bei 4 °C gelagert, wohingegen die Stammlösung durch Lösen der Reinsubstanz in Ethanol (100 mg/ml), gefolgt von einer endgültigen Verdünnung mit "DMEM High-Glucose" (Biochrom, Berlin, Deutschland; 1 mg/ml), hergestellt wird.

### MTT-Test

Die Zellproliferation wird mittels eines quantitativen kolorimetrischen 3-(4,5-Dimethylthiazol-2-yl)-2,5-diphenyltetrazoliumbromid-basierten (MTT) Zelltests bestimmt. Dieser Test bestimmt die Anzahl der lebensfähigen Zellen auf Basis der mitochondrialen Konversion von MTT.

Dazu werden 5.000 Zellen in jedes Well einer 96-Well-Platte verteilt. Nach 24 Stunden Kultivierung werden vier verschiedene Konzentrationen von 5-Fluoruracil oder Cisplatin zu den Kulturen hinzugefügt oder die Zellen werden mit drei Dosen bestrahlt. Nach 48 Stunden werden in jedes Well 10 µl MTT-Farbstoff (5 mg/ml) zugefügt. Nach zwei Stunden Inkubation mit MTT wurden die Kristalle gelöst und vorsichtig für 24 Stunden bei Raumtemperatur geschüttelt. Mithilfe eines Multi-Well-ELISA-Lesegeräts wird die Absorption der reduzierten Formazan-Produkte in Kontrolle und Proben-Wells bei einer Welle von 570 nm und 690 nm bestimmt. Alle Wachstumsproben des MTT-Tests werden in dreifacher Ausführung ausgeführt.

### 3. Ergebnisse

### Verringerte β-Catenin-Aktivität als Reaktion auf die Behandlung mit 1,8-Cineol

Um den Einfluss von 1,8-Cineol auf die Aktivität und Regulation der Wnt/β-Catenin-Signalkaskade zu untersuchen, wird zunächst das Expressionslevel von aktivem β-Catenin, bei welchem es sich um ein zentrales transkriptionelles Aktivatorprotein dieses Signalwegs handelt, analysiert. Die Ergebnisse zeigen im Allgemeinen eine starke Abnahme von aktivem β-Catenin als Reaktion auf eine 24-stündige Inkubation mit 100 µM 1,8-Cineol in den analysierten HNSCC Zelllinien im Vergleich zur Mediumkontrolle. Diese Daten zeigen eine Inhibition des Wnt/β-Catenin-Signalwegs in HNSCC als Folge der Behandlung mit 1,8-Cineol (Fig. 1A).

### Regulation von GSK3 als Reaktion auf 1,8-Cineol

In einem nächsten Schritt werden weitere Untersuchungen durchgeführt, um die korrespondierenden Phosphorylierungslevel des β-Catenin-Regulators GSK-3 zu untersuchen. Bei GSK-3 handelt es sich um eine konstitutiv aktive Proteinkinase, welche durch die Phosphorylierung an Serin-9 (GSK-3α) oder Serin-21 (GSK-3β) inhibiert wird. Western Blot-Experimente werden durchgeführt, um die Expression und Phosphorylierungslevel von GSK-3α/β in Anwesenheit und Abwesenheit von 100 µM 1,8-Cineol zu untersuchen.

Die seitens der Anmelderin gewonnenen Daten zeigen eindeutig, dass Cineol zu einem verringerten Level von bzw. Gehalt an phosphoryliertem GSK-3α/β (Serin-9/-21) führt, was in der Folge zu einer verstärkten Aktivität von GSK-3 und somit zu einer verstärkten Degradation von β-Catenin führt (Fig. 1B). Die Phosphorylierung von Tyrosin-279 in GSK-3α und Tyrosin-216 in GSK-3β führt zu einer verstärkten GSK-3-Aktivität. Wie Fig. 1C zeigt, werden die Expressionslevel von GSK-3α/β (Tyrosin-279/-216) durch 1,8-Cineol nicht nennenswert beeinflusst.

Zusätzlich zu der Phosphorylierung an Serin-9/-21, wie zuvor definiert, wird die GSK-3-Aktivität auch auf dem Level der subzellulären Lokalisierung reguliert. Um die Kinase-Aktivität zu verringern, kann GSK-3 in endolysosomale Vesikel umgelagert werden, um von seinen Substraten getrennt zu werden. Die diesbezüglich durchgeführten immunohistochemischen Untersuchungen zeigen eine verringerte endolysosomale Lokalisation von GSK-3 als Reaktion auf 100 µM 1,8-Cineol, was dessen verringerte Inhibition nach Behandlung mit 1,8-Cineol unterstützt (Fig. 2).

GSK-3 ist in die Regulation zahlreicher biosynthetischer Wege involviert und zahlreiche Kinasen sind in der Lage, die GSK-3-Aktiviät mittels Phosphorylierung an dessen aktivierenden oder inhibierenden Phosphorylierungsstellen zu regulieren. Die Akt-Kinase (auch bekannt als PKB) wurde im Stand der Technik als ein Onkogen mit Serin-/Threonin-Kinase-Aktivität identifiziert, und es wurde gezeigt, dass sie ein negativer Regulator für die GSK-3β-Aktivität *in vivo* ist. Akt wird dabei durch die Bindung von Phospholipiden aktiviert und die Aktivierungsschleife mittels Phosphorylierung von Threonin-308 durch PDK1. Vor diesem Hintergrund wird vorliegend in einem ersten Ansatz die Expressions- und Phosphorylierungslevel von Akt als Reaktion auf 100 µM 1,8-Cineol analysiert. Weder die Expression noch die Phosphorylierung von Threonin-308 von Akt wird in den analysierten Zelllinien nennenswert beeinflusst (Fig. 3).

### Verringerte Wnt11-Expression als Reaktion auf 1,8-Cineol

Weiterhin werden im Hinblick auf die Wnt/β-Catenin-Signalgebung aufgrund ihrer Involvierung in die Regulation der epithelial-mesenchymalen Transition (EMT) die transkriptionellen Profile verschiedener Gene, welche in Zusammenhang mit EMT stehen, in Reaktion auf 1,8-Cineol untersucht. Dafür wird der PCR-basierte "RT² EMT Profiler" eingesetzt, welcher die umfassende Analyse von 84 verschiedenen, in Zusammenhang mit EMT stehenden Genen erlaubt. Die erhaltenen Daten zeigten einen starken Rückgang der mRNA-Level von Wnt11 als Reaktion auf 100 µM 1,8-Cineol in den analysierten HNSCC Zelllinien.

Um dieses Ergebnis auf Proteinebene zu bestätigen, werden sechs verschiedene Zelllinien von HNSCC mittels Western Blot-Experimente analysiert, wobei ein spezifischer Anti-Wnt11-Antikörper eingesetzt wird. Es kann gezeigt werden, dass die Proteinexpression von Wnt11 dosisabhängig in Reaktion auf 1,8-Cineol abnimmt. Die analysierten permanenten HNSCC Zelllinien 16A, SCC154, 60B und Carey24 zeigen eine starke Herunterregulation von Wnt11 nach 24 Stunden Behandlung mit 1mM Cineol. Die Zelllinien 16B und 60A zeigen gleichermaßen einen Rückgang der Wnt11 Proteinexpression, auch wenn dieser geringer ist (Fig. 4).

Dosisabhängige Inhibition der Proliferation von HNSCC durch 1,8-Cineol Um zu bestimmen, ob die Behandlung mit 1,8-Cineol zu einer Inhibition der zellulären Lebensfähigkeit von HNSCC führt, werden MTT-Tests durchgeführt. Zu diesem Zweck werden die Zellen für 288 Stunden in 300 µM bzw. 700 µM Cineol inkubiert.

Als Reaktion auf die Behandlung mit 700 µM Cineol kann in den analysierten permanenten Zelllinien ein starker Rückgang der Proliferation und zellulären Lebensfähigkeit von HNSCC-Zellen beobachtet werden (Fig. 5).

### Einfluss von Cineol auf Zellvitalität und Proliferation

Es werden weiterführende Untersuchungen durchgeführt zur Analyse des Einflusses von Cineol auf die Vitalität und Proliferation verschiedener permanenter Zelllinien. Hierzu werden einerseits die zwei HNSCC-Zelllinien und des Weiteren eine Fibroblastenzelllinie verwendet. Diese werden über einen Zeitraum von 250 Stunden mit verschiedenen Cineol-Konzentrationen kultiviert (1 mM bis 10 mM) und die Vitalität der Zellen wird jeweils mittels eines MTT-Assays untersucht. Es wird sowohl ein inhibierender Einfluss von Cineol auf die Progression und Vitalität von HNSCC als auch ein inhibierender Einfluss von Cineol auf die Progression und Vitalität von Fibroblasten gefunden.

Diese Untersuchungen unterstreichen deutlich die beobachtete starke proliferationshemmende Wirkung von Cineol auch ab einer Konzentration von 1mM bis 2mM und die deutlich zytotoxische Wirkung bei 10mM in den untersuchten Zellspezies.

Die Untersuchungen zeigen den Einfluss von Cineol auf Zellviabilität und Progression bei malignen Zellspezies.

### 4. Diskussion

Bei dem Monoterpen-Oxid 1,8-Cineol handelt es sich um den natürlichen pflanzlichen Wirkstoff des klinisch zugelassenen Arzneimittels Soledum®, welches zur Therapie von Erkrankungen der Atemwege, wie chronischer Sinusitis, Bronchitis oder Asthma, verwendet wird. 1,8-Cineol wird im Stand der Technik als antibakterieller und antiinflammatorischer Wirkstoff eingesetzt.

Die vorliegenden Ergebnisse zeigen demgegenüber nunmehr erstmals eine neue Wirkweise von 1,8-Cineol durch die Inhibition des Wnt/β-Catenin-Signalwegs in HNSCC, welche zu einem verringerten Expressionslevel von Wnt11 und zu einer verringerten zellulären Progression führt. Dies führt zu einem neuen therapeutischen Konzept.

Bei den Liganden von Wnt handelt es sich um eine große Familie sekretierter Proteine, welche eine wichtige Rolle in einer Vielzahl von Prozessen, wie der Embryonalentwicklung, Zellproliferation oder Zellpolarität, spielen. Üblicherweise werden die Signale von Wnt in zwei Typen klassifiziert, nämlich den kanonischen (β-Catenin-abhängigen) bzw. den nicht-kanonischen (β-Cateninunabhängigen) Typ. Diese Klassifizierung basiert auf der biologischen Aktivität, wobei kanonische Wnts (z. B. Wnt 1 und Wnt 3a) die Ausbildung einer Zweitachse in Embryonen von *Xenopus laevis* induzieren, während nichtkanonische Wnts (z. B. Wnt 4, Wnt 5a und Wnt 11) dies nicht bewirken. Tatsächlich ist die Wnt-Signalgebung deutlich komplexer und kontextabhängig. Es sind zahlreiche interzelluläre regulatorische Kaskaden bekannt, von denen einige sowohl aus kanonischen als auch nicht-kanonischen Komponenten bestehen.

Zusammenfassend zeigen die zuvor in Abschnitt 3.) angeführten Ergebnisse einen neuen Therapieansatz und Wirkeffekt für die 1,8-Cineol-Aktivität, und zwar die Inhibition des Wnt/β-Catenin-Signalwegs insbesondere in HNSCC, welche zu einem verringerten Expressionslevel von Wnt11 und zu einer verringerten zellulären Progression führt, so dass erfindungsgemäß auf dieser Basis ein neuer Behandlungsansatz von Cineol als natürlichen bzw. naturbasierten Wirkstoff bereitgestellt wird.

Im Rahmen dieser Arbeit hat die Anmelderin somit erstmals gezeigt, dass 1,8-Cineol als starker Inhibitor der Wnt/β-Catenin-Aktivität fungiert und dabei zu einem starken Rückgang der zellulären Progression führt.

Die verringerte Degradation von β-Catenin korreliert mit einer beeinflussten bzw. modulierten bzw. gesteuerten Inhibition von GSK3, insbesondere von GSK3-β, wobei die für diesen Mechanismus verantwortlichen angesprochenen Proteine nicht weiter aufgeklärt worden sind (vgl. Figur 6 als Modell).

Zusammenfassend lässt sich feststellen, dass die Ergebnisse der Anmelderin ein neues Therapiekonzept für die 1,8-Cineol-Aktivität aufzeigen und zu neuen Behandlungsanwendungen dieses natürlichen Arzneimittels führen. Zusammenfassend kann also im Rahmen der vorliegenden Erfindung erstmals ein neuartiges, zumindest im Wesentlichen nebenwirkungsfreies Therapiekonzept für die prophylaktischen und/oder therapeutische Behandlung von insbesondere malignen Tumorerkrankungen und/oder Karzinomerkrankungen des Kopf-Hals-Bereichs auf Basis eines naturbasierten Wirkstoffs (Cineol, insbesondere 1,8-Cineol) bereitgestellt werden, mit welchem die Nachteile des Standes der Technik zumindest weitgehend vermieden oder aber wenigstens abgeschwächt werden können und mit welchem eine hervorragende Wirkeffizienz im Vergleich bzw. in Ergänzung zu den Therapiekonzepten des Standes der Technik erreicht werden kann.

## Patentansprüche

1. Zusammensetzung, insbesondere pharmazeutische Zusammensetzung, zur Verwendung bei der prophylaktischen und/oder therapeutischen Behandlung von malignen Tumorerkrankungen und/oder Karzinomerkrankungen des Kopf-Hals-Bereichs,
wobei die Tumorerkrankungen und/oder Karzinomerkrankungen des Kopf-Hals-Bereichs ausgewählt sind aus Kopf-Hals-Plattenepithel-Karzinomen (HNSCC),
wobei die Zusammensetzung als alleinigen Wirkstoff 1,8-Cineol enthält,
wobei das Cineol eine Reinheit von mindestens 95 Gew.-%, bezogen auf das Cineol, aufweist und wobei das Cineol frei von anderen Terpenen ist.

2. Zusammensetzung zur Verwendung nach Anspruch 1, wobei die Tumorerkrankungen und/oder Karzinomerkrankungen des Kopf-Hals-Bereichs ausgewählt sind aus der Gruppe von Kopf-Hals-Plattenepithel-Karzinomen (HNSCC) der Mundhöhle, des Mundrachens, der inneren Nase, der Nasennebenhöhle und des Nasenrachens.

3. Zusammensetzung zur Verwendung nach Anspruch 1 oder 2, wobei die Kopf-Hals-Plattenepithel-Karzinome (HNSCC) ausgewählt sind aus der Gruppe von Mundhöhlenkarzinomen, Oropharynxkarzinomen, Nasenhöhlenkarzinomen, Nasopharynxkarzinomen, Hypopharynxkarzinomen, Larynxkarzinomen und Trachealkarzinomen, insbesondere aus der Gruppe von Mundhöhlenkarzinomen, Oropharynxkarzinomen, Nasenhöhlenkarzinomen und Nasopharynxkarzinomen.

4. Zusammensetzung zur Verwendung nach einem der vorangehenden Ansprüche, wobei die Zusammensetzung zur prophylaktischen und/oder therapeutischen Behandlung von postoperativen Zuständen eingesetzt wird.

5. Zusammensetzung zur Verwendung nach einem der vorangehenden Ansprüche, wobei die Zusammensetzung zur prophylaktischen und/oder therapeutischen Behandlung der Kopf-Hals-Plattenepithel-Karzinome (HNSCC) nach vorangehender chirurgischer Entfernung von Tumoren eingesetzt wird.

6. Zusammensetzung zur Verwendung nach einem der vorangehenden Ansprüche, wobei das Cineol eine Reinheit von mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das Cineol, aufweist.

7. Zusammensetzung zur Verwendung nach einem der vorangehenden Ansprüche, wobei die Zusammensetzung das Cineol in wirksamen, insbesondere pharmazeutisch wirksamen Mengen enthält.

8. Zusammensetzung zur Verwendung nach einem der vorangehenden Ansprüche, wobei die Zusammensetzung das Cineol, bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

9. Zusammensetzung zur Verwendung nach einem der vorangehenden Ansprüche, wobei das Cineol mit einer Tagesdosis im Bereich von 0,1 bis 5.000 mg/diem, insbesondere im Bereich von 1 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird.

10. Zusammensetzung zur Verwendung nach einem der vorangehenden Ansprüche,
wobei das Cineol systemisch mit einer Tagesdosis im Bereich von 10 bis 5.000 mg/diem, insbesondere im Bereich von 50 bis 3.000 mg/diem, vorzugsweise im Bereich von 100 bis 2.500 mg/diem, bevorzugt im Bereich von 150 bis 2.000 mg/diem, besonders bevorzugt im Bereich von 200 bis 1.500 mg/diem, verabreicht wird oder aber
wobei das Cineol topisch mit einer Tagesdosis im Bereich von 0,1 bis 2.000 mg/diem, insbesondere im Bereich von 0,5 bis 1.500 mg/diem, vorzugsweise im Bereich von 1 bis 1.000 mg/diem, bevorzugt im Bereich von 2 bis 1.500 mg/diem, besonders bevorzugt im Bereich von 5 bis 1.000 mg/diem, verabreicht wird.

11. Zusammensetzung zur Verwendung nach einem der vorangehenden Ansprüche, wobei die Zusammensetzung als Kombinationstherapeutikum mit Onkoziden, insbesondere ausgewählt aus der Gruppe von Antitumormitteln, Chemotherapeutika und Cytostatika, und/oder mit Strahlentherapie eingesetzt wird.

12. Arzneimittel zur Verwendung bei der prophylaktischen und/oder therapeutischen Behandlung von malignen Tumorerkrankungen und/oder Karzinomerkrankungen des Kopf-Hals-Bereichs, wobei die Tumorerkrankungen und/oder Karzinomerkrankungen des Kopf-Hals-Bereichs ausgewählt sind aus Kopf-Hals-Plattenepithel-Karzinomen (HNSCC),
wobei das Arzneimittel als alleinigen Wirkstoff 1,8-Cineol enthält,
wobei das Cineol eine Reinheit von mindestens 95 Gew.-%, bezogen auf das Cineol, aufweist und wobei das Cineol frei von anderen Terpenen ist.

13. Wirkstoffkombination zur Verwendung bei der prophylaktischen und/oder therapeutischen Behandlung von malignen Tumorerkrankungen und/oder Karzinomerkrankungen des Kopf-Hals-Bereichs, wobei die Tumorerkrankungen und/oder Karzinomerkrankungen des Kopf-Hals-Bereichs ausgewählt sind aus Kopf-Hals-Plattenepithel-Karzinomen (HNSCC),
wobei die Wirkstoffkombination als räumlich getrennte, aber funktional zusammenhängende Komponenten
(a) 1,8-Cineol in Form einer Cineol als alleinigen Wirkstoff enthaltenden, topisch oder systemisch zu applizierenden pharmazeutischen Zusammensetzung, wobei das Cineol eine Reinheit von mindestens 95 Gew.-%, bezogen auf das Cineol, aufweist und wobei das Cineol frei von anderen Terpenen ist, einerseits und
(b) mindestens ein Onkozid, ausgewählt aus der Gruppe von Antitumormitteln, Chemotherapeutika und Cytostatika, in Form einer das Onkozid enthaltenden, topisch oder systemisch zu applizierenden pharmazeutischen Zusammensetzung,
umfasst.

## Claims

1. A composition, in particular a pharmaceutical composition, which is suitable for use in the prophylactic and/or therapeutic treatment of malignant tumour diseases and/or carcinogenic diseases of the head and neck region,
wherein the tumour diseases and/or carcinogenic diseases of the head and neck region are selected from head and neck squamous cell carcinomas (HNSCC),
wherein the composition contains 1,8-cineole as the sole active substance,
wherein the cineole has a purity of at least 95 wt%, in relation to the cineole, and wherein the cineole is free of other terpenes.

2. The composition for use according to claim 1, wherein the tumour diseases and/or carcinogenic diseases in the head and neck region are selected from the group of head and neck squamous cell carcinomas (HNSCC) of the oral cavity, the oropharynx, the inner nose, the paranasal sinus and the nasopharynx.

3. The composition for use according to claim 1 or 2, wherein the head and neck squamous cell carcinomas (HNSCC) are selected from the group of oral carcinomas, oropharyngeal carcinomas, nasal carcinomas, nasopharyngeal carcinomas, hypopharyngeal carcinomas, laryngeal carcinomas and tracheal carcinomas, in particular from the group of oral carcinomas, oropharyngeal carcinomas, nasal carcinomas and nasopharyngeal carcinomas.

4. The composition for use according to any one of the preceding claims, wherein the composition is used for the prophylactic and/or therapeutic treatment of postoperative conditions.

5. The composition for use according to any one of the preceding claims, wherein the composition is used for the prophylactic and/or therapeutic treatment of head and neck squamous cell carcinoma (HNSCC) after prior surgical removal of tumours.

6. The composition for use according to any one of the preceding claims, wherein the cineole has a purity of at least 96 wt%, preferably at least 97 wt%, particularly preferably at least 98 wt%, very particularly preferably at least 99 wt%, even more preferably at least 99.5 wt%, relative to the cineole.

7. The composition for use according to any one of the preceding claims, wherein the composition contains the cineole in effective, in particular pharmaceutically effective amounts.

8. The composition for use according to any one of the preceding claims, wherein the composition contains the cineole, in relation to the composition, in relative amounts in the range of 0.0001 to 80 wt%, in particular 0.001 to 75 wt%, preferably 0.005 to 70 wt%, preferably 0.01 to 60 wt%, particularly preferably 0.05 to 55 wt%, very particularly preferably 0.1 to 50 wt%.

9. The composition for use according to any one of the preceding claims, wherein the cineole is administered with a daily dose in the range of 0.1 to 5000 mg/diem, in particular in the range of 1 to 3000 mg/diem, preferably in the range of 5 to 2500 mg/diem, more preferably in the range of 10 to 2000 mg/diem, particularly preferably in the range of 50 to 1500 mg/diem.

10. The composition for use according to any one of the preceding claims,
wherein the cineole is administered systematically with a daily dose in the range of 10 to 5000 mg/diem, in particular in the range of 50 to 3000 mg/diem, preferably in the range of 100 to 2500 mg/diem, more preferably in the range of 150 to 2000 mg/diem, particularly preferably in the range of 200 to 1500 mg/diem, or
wherein the cineole is administered topically with a daily dose in the range of 0.1 to 2000 mg/diem, in particular in the range of 0.5 to 1500 mg/diem, preferably in the range of 1 to 1000 mg/diem, more preferably in the range of 2 to 1500 mg/diem, particularly preferably in the range of 5 to 1000 mg/diem.

11. The composition for use according to any one of the preceding claims, wherein the composition is used as a combination therapy with oncocidals, in particular selected from the group of anti-tumour agents, chemotherapeutic agents and cytostatics, and/or with radiation therapy.

12. A medicinal product for use in the prophylactic and/or therapeutic treatment of malignant tumour diseases and/or carcinogenic diseases of the head and neck region, wherein the tumour diseases and/or carcinogenic diseases of the head and neck region are selected from head and neck squamous cell carcinomas (HNSCC),
wherein the medicinal product contains 1,8-cineole as the sole active substance,
wherein the cineole has a purity of at least 95 wt%, in relation to the cineole, and wherein the cineole is free of other terpenes.

13. An active substance combination for use in the prophylactic and/or therapeutic treatment of malignant tumour diseases and/or carcinogenic diseases of the head and neck region, wherein the tumour diseases and/or carcinogenic diseases of the head and neck region are selected from head and neck squamous cell carcinomas (HNSCC),
wherein the active substance combination as spatially separated but functionally related components
(a) comprises 1,8-cineole in the form of a pharmaceutical composition which contains cineole as the sole active substance and which is to be administered topically or systemically, wherein the cineole has a purity of at least 95 wt%, in relation to the cineole, and wherein the cineole is free of other terpenes, on the one hand, and
(b) comprises at least one oncocidal, selected from the group of anti-tumour agents, chemotherapeutic agents and cytostatics, in the form of a pharmaceutical composition which contains the oncocidal and which is to be administered topically or systemically.

## Revendications

1. Composition, en particulier composition pharmaceutique, à utiliser pour le traitement prophylactique et/ou thérapeutique de maladies tumorales malignes et/ou de maladies cancéreuses de la zone de la tête et du cou,
les maladies tumorales et/ou maladies cancéreuses de la zone de la tête et du cou étant choisies parmi les carcinomes épidermoïdes de la tête et du cou (HNSCC),
la composition contenant du 1,8-cinéol en tant que seul principe actif,
le cinéol ayant une pureté d'au moins 95 % en poids, par rapport au cinéol et le cinéol étant exempt d'autres terpènes.

2. Composition à utiliser selon la revendication 1, les maladies tumorales et/ou les maladies cancéreuses de la zone de la tête et du cou étant choisies dans le groupe des carcinomes épidermoïdes de la tête et du cou (HNSCC) de la cavité buccale, de l'oropharynx, du nez interne, du sinus paranasal et du nasopharynx.

3. Composition à utiliser selon la revendication 1 ou 2, les carcinomes épidermoïdes de la tête et du cou (HNSCC) étant choisis dans le groupe des carcinomes de la cavité buccale, des carcinomes de l'oropharynx, des carcinomes de la cavité nasale, des carcinomes du nasopharynx, des carcinomes de l'hypopharynx, des carcinomes du larynx et des carcinomes trachéens, en particulier dans le groupe des carcinomes de la cavité buccale, des carcinomes de 'oropharynx, des carcinomes de la cavité nasale et des carcinomes du nasopharynx.

4. Composition à utiliser selon l'une des revendications précédentes, la composition étant utilisée pour le traitement prophylactique et/ou thérapeutique d'affections postopératoires.

5. Composition à utiliser selon l'une des revendications précédentes, la composition étant utilisée pour le traitement prophylactique et/ou thérapeutique du carcinome épidermoïde de la tête et du cou (HNSCC) après l'ablation chirurgicale préalable de tumeurs.

6. Composition à utiliser selon l'une des revendications précédentes, le cinéol ayant une pureté d'au moins 96 % en poids, de préférence d'au moins 97 % en poids, de manière particulièrement préférée d'au moins 98 % en poids, le plus préférablement d'au moins 99 % en poids, toujours plus préférablement au moins 99,5 %, par rapport au cinéol.

7. Composition à utiliser selon l'une des revendications précédentes, la composition contenant le cinéol en des quantités efficaces, en particulier pharmaceutiquement efficaces.

8. Composition à utiliser selon l'une des revendications précédentes, la composition contenant le cinéol, par rapport à la composition, en des quantités relatives situées dans la plage allant de 0,0001 à 80 % en poids, en particulier de 0,001 à 75 % en poids, de préférence de 0,005 à 70 % en poids, de manière plus préférée de 0,01 à 60 % en poids, de manière particulièrement préférée de 0,05 à 55 % en poids, le plus préférablement de 0,1 à 50 % en poids.

9. Composition à utiliser selon l'une des revendications précédentes, le cinéol étant administré à une dose quotidienne située dans la plage allant de 0,1 à 5000 mg/jour, en particulier dans la plage allant de 1 à 3000 mg/jour, de préférence dans la plage allant de 5 à 2500 mg/jour, de manière plus préférée dans la plage allant de 10 à 2000 mg/jour, de manière particulièrement préférée dans la plage allant de 50 à 1500 mg/jour.

10. Composition destinée à être utilisée selon l'une des revendications précédentes,
le cinéol étant administré par voie générale à une dose journalière située dans la plage allant de 10 à 5000 mg/jour, en particulier dans la plage allant de 50 à 3000 mg/jour, de préférence dans la plage allant de 100 à 2500 mg/jour, de manière plus préférée dans la plage allant de 150 à 2000 mg/jour, de manière particulièrement préférée dans la plage allant de 200 à 1500 mg/jour, ou cependant
le cinéol étant administré par voie topique à une dose journalière située dans la plage allant de 0,1 à 2000 mg/jour, en particulier dans la plage allant de 0,5 à 1500 mg/jour, de préférence dans la plage allant de 1 à 1000 mg/jour, de manière plus préférée dans la plage allant de 2 à 1500 mg/jour, de manière particulièrement préférée dans la plage allant de 5 à 1000 mg/jour.

11. Composition à utiliser selon l'une des revendications précédentes, la composition étant utilisée en association thérapeutique avec des oncosides, en particulier choisis dans le groupe des agents antitumoraux, des agents chimiothérapeutiques et des cytostatiques et/ou avec une radiothérapie.

12. Médicament à utiliser pour le traitement prophylactique et/ou thérapeutique de maladies cancéreuses et/ou de maladies tumorales malignes de la zone de la tête et du cou, les maladies tumorales et/ou les maladies cancéreuses de la zone de la tête et du cou étant choisies parmi les carcinomes épidermoïdes de la tête et du cou (HNSCC),
le médicament contenant du 1,8-cinéol comme seul principe actif,
le cinéol ayant une pureté d'au moins 95 % en poids, par rapport au cinéol et le cinéol étant exempt d'autres terpènes.

13. Association de médicaments à utiliser pour le traitement prophylactique et/ou thérapeutique de maladies cancéreuses et/ou de maladies tumorales malignes de la zone de la tête et du cou, les maladies tumorales et/ou les maladies cancéreuses de la zone de la tête et du cou étant choisies parmi les carcinomes épidermoïdes de la tête et du cou (HNSCC),
l'association de médicaments présentant, en tant que composants spatialement séparés mais fonctionnellement liés
(a) du 1,8-cinéol sous la forme d'une composition pharmaceutique contenant du cinéol comme seul principe actif, à administrer par voie topique ou générale, le cinéol ayant une pureté d'au moins 95 % en poids, par rapport au cinéol et le cinéol étant exempt d'autres terpènes d'une part et
(b) au moins un oncoside choisi dans le groupe des agents antitumoraux, des agents chimiothérapeutiques et des cytostatiques, sous la forme d'une composition pharmaceutique contenant un oncoside, à administrer par voie topique ou systémique.
